(19) Europäisches Patentamt
European Patent Office
Office européen des brevets

(11) **EP 2 077 759 B1**

(12) **EUROPEAN PATENT SPECIFICATION**

(45) Date of publication and mention
of the grant of the patent:
**26.07.2017 Bulletin 2017/30**

(51) Int Cl.:
*A61B 8/00* *(2006.01)* *A61B 8/08* *(2006.01)*
*A61B 8/13* *(2006.01)* *A61B 5/00* *(2006.01)*

(21) Application number: **07827257.2**

(22) Date of filing: **24.10.2007**

(86) International application number:
**PCT/IL2007/001283**

(87) International publication number:
**WO 2008/050333 (02.05.2008 Gazette 2008/18)**

(54) **3D QUANTITATIVE ULTRASONIC DEVICE FOR BONE INSPECTION AND ITS IMPLEMENTATION**

QUANTITATIVE 3D-ULTRASCHALLVORRICHTUNG ZUR KNOCHENINSPEKTION

DISPOSITIF ULTRASONORE D'IMAGERIE 3D QUANTITATIF POUR INSPECTION OSSEUSE

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **24.10.2006 US 853759 P**

(43) Date of publication of application:
**15.07.2009 Bulletin 2009/29**

(73) Proprietors:
• **Mindeles, Gesel
35591 Haifa (IL)**
• **Gourevitch, Alla
33072 Haifa (IL)**

(72) Inventor: **GOUREVITCH, Alla
33072 Haifa (IL)**

(74) Representative: **Taor, Simon Edward William
Venner Shipley LLP
200 Aldersgate
London EC1A 4HD (GB)**

(56) References cited:
**US-A1- 2002 161 300     US-A1- 2005 283 072
US-B1- 6 221 019     US-B1- 6 432 057**

## Description

FIELD OF THE INVENTION

The present invention relates to a quantitative-imaging ultrasound technology and equipment.

BACKGROUND OF THE INVENTION

Significant numbers of ultrasonic methods exist, which provide imaging soft tissues of a human body. They are based for example on Compound Imaging approach, Stereoscopic imaging, Harmonic imaging, Spatial Compounding Approach and others methods. These methods are disclosed for example in the patents: U.S. Patent 6,517,487; U.S. Patent Applications 20030055334, 20030055337, 20040193047, 20050033140, 20050049479, 20050124886, 20050117694, and others.

Ultrasonic imaging methods known in the art are unsuitable for assessing the skeleton of a human body and therefore the conventional ultrasonic imaging systems are used for studying soft tissue and are not optimized for assessing the skeleton.

One of the reasons for the unsuitability of ultrasonic imagers to perform bone studies is a high attenuation coefficient of a bone compared with a soft tissue.

Ultrasonic methods for simultaneously quantitative and imaging measurements are known in the art.

For example, United States Patent Application 20030018263; to Morris, et al; entitled Multi-Zone Transmitter For Quantitative Ultrasound And Image Measurement; filed: July 20, 2001; discloses an ultrasonic transmission unit for an imaging/quantitative ultrasound device provides for coaxial transducer crystals which may be operated independently with a first crystal operated alone for quantitative measurement and the first and second crystal operated together to provide a broad illumination for imaging of structure.

U.S. Patent Application 20050107700 proposes a thin film piezoelectric material employs a metallic backer plate to provide high output, non-resonant ultrasonic transmissions suitable for quantitative ultrasonic measurement and/or imaging. Thin film polymer piezoelectric materials such as polyvinylidene fluoride (PDVF) may also be used as an ultrasonic transducer as described in the U.S. Patents No. 6,305,060 and U.S. Patent No. 6,012,779.

However, the disclosed methods do not provide 3D imaging considered objects.

Ultrasonic investigations of skeleton can be carried out with the aim of the Quantitative UltraSonography (QUS) methods. The main ultrasonic parameters evaluated in QUS measurement are the Speed of Sound (SOS) and the frequency dependence of attenuation.

Ultrasonic Attenuation (BUA) technique is described by Langton C M, Palmer S B, Porter R W, The Measurement of Broadband Ultrasonic Attenuation in Trabecular Bone, Engineering in Medicine, 13 3, 89-91 (1984). Whilst it now forms the basis of clinical QUS bone assessment, this empirical method is not entirely satisfactory. The BUA technique essentially measures insertion loss, found by comparing the amplitude spectrum of an ultrasonic pulse through bone with that through a reference medium, typically water. The assumption is made that the attenuation, a (f), is a linear function of frequency, f, between 200 - 600 kHz. However, a number of authors argued that the assumption of linear relationship does not have any physical basis. (Elinor R Hughes MIOA, Timithy G Leighton FIOA, Graham W Petley, Paul R White "Ultrasonic assessment of bone health" (http://www.isrt.soton.ac.uk)

United States Patent 6,371,916; entitled Acoustic Analysis of Bone Using Point-Source-Like Transducers; to Buhler, et al.; filed: September 3, 1999; discloses an improved apparatus and method for providing a measurement of the characteristic behavior of an acoustic wave in a bone of a subject. A preferred embodiment has first and second transducers and a mounting arrangement for mounting the transducers in spaced relationship with respect to the bone. The first transducer may transmit acoustic energy over a broad solid angle, thereby behaving as a point source of acoustic energy. Additionally or alternatively, the second transducer may collect acoustic energy over a broad solid angle, thereby behaving as a point receiver. A signal processor in communication with the second transducer provides a measurement that is a function of at least one of transient spectral or transient temporal components of the signal received by the second transducer.

The disclosed invention provides determining an index of porosity (indirect qualitative but not quantitative parameter of porosity estimation) and non-connectivity of a bone, but the method not allows obtain 3D imaging of bones in a human body. It does not provide images of porosity values distributions in a considered volume.

The oil geophysics proposes the method for determining porosity of inspected media. The invention uses modified Wyllie relationship which can be found in Wyllie, M.R., An experimental investigation of factors affecting elastic wave velocities in porous media, Geophysics, vol. 23, No. 3, 1958. But this approach does not provide volume distribution of obtained porosity in an inspected object.

An acoustic energy may travel, reflect, and refract on a boundary between media with different acoustic impedances.

Traveling acoustic energy is used in the direct transmission method. For example the U.S. Patents 4,926,870; 4,421,119 describe systems, which place a receiver and a transmitter on opposite sides of a bone. The linear propagation of elastic waves is used in the method. It is conventional method for bone tissues inspections. The method provides integral estimations. An obtained data characterize integral estimation about travel longitudinal waves from transmitter to receiver in heterogeneous object, specifically bones. Measured parameters are increased from one part of an investigated object and are reduced from another it's a part in the case investigations of heterogeneous substances. Significant mistakes are obtained in results; because the method not sensitive to an investigated object structure changes and it ignores a medium heterogeneous and anisotropy.

Inspection methods based on refracted energy are used in industry. For example, Time of Flight Diffraction technique (TOFD) is disclosed U.S. Patent Application 20020134178; to Knight, et al.; entitled Ultrasonic Testing System.

In this method a probe with different beam angles is used to detect planar defects through varying an angle of orientation. Usually two probes, one transmitter and one receiver, are arranged on an object surface. The transmitter sends a relatively wide beam for maximizing the field of the scan. Both probes are aligned geometrically on either side of a considered object and an A-scan taken at a sequential position along the length of the object. The data collection on site by the TOFD method is faster than most conventional methods because it uses wide ultrasonic beams for imaging. It is a technique for precise depth assessment. However, this technique does not apply for investigations anisotropic and heterogeneous materials and is not used in medicine.

Contrary to X-ray CT, Ultrasonic tomography of elastic wave velocities has not found general practical usage in the medical imaging. The main problems are gas and bone inclusions and associated deviation of the ultrasound beam due to reflection and refraction because the systems are heterogeneous media.

The use of elastic wave refraction for investigations bone structures of a human body for medical diagnostics is described in the U.S. Patents No. 6,221,019 and U.S. Patents No. 5,143,072. The disclosed methods assume that the refracted waves travel in the field with a boundary between layers with different acoustic impedance. It is the boundary between tissue and bone.

In the disclosed patents, a transmitter and a receiver are placed on the skin of a patient facing to a bone. Ultrasonic waves are transmitted along a transmission path from the transmitter to the bone through the soft tissue surrounding the bone, along the surface of the bone and back through the soft tissue to the receiver location 1 and location 2. The travel times of the fastest signals between the transmitter and receiver (for location 1 and for location2) are measured and the acoustic velocity of the bone is calculated based on the distance between the transmitter and the receiver's two locations, the thickness of the soft tissue and the acoustic velocity in the soft tissue. The reflected waves are used for estimations of the acoustic velocity and thickness of soft tissues. The measured parameter, Speed Of Sound (SOS) of a cortical bone is calculated from the obtained measured data.

However, this method has the several serious shortcomings.

- The method is based only on two layers soft tissues and bone in its algorithm
- The method cannot account for anisotropy and heterogeneous nature of bone's media and thus produces an integral estimation that lead to significant mistakes and to insensitive of the method (inspected parameter increased from one part of an inspected bone and at the same time reduced from another its a part).

  - It does not map the distribution of structure's changes in a bone by means of the estimated parameters distribution in the bone - heterogeneous medium.

- The method only evaluates the bone medium unknown and changeable by depth from quality observed bone upper cortical layer integral estimations and only in single direction.

  - The method does not provide a 3D imaging of the inspected bone volume.
  - The method suffers an additional inaccuracy and inconvenience by the necessity to measure a thickness and a longitudinal wave velocity in soft tissues by applying an additional Pulse-Echo method.

- The method does not estimate the mineral part (matrix) of an inspected bone and porosity values also as porosity distributions in a bone's volume.
- The method requires the referent data for fracture risk estimations.
- The method does not provide location and estimation fracture risk.

SUMMARY OF THE INVENTION

According to an aspect of the present invention, there is provided an imaging ultrasound tomography system according to claim 1. Further aspects of the imaging ultrasound tomography system are provided according to dependent claims 2 - 8.

BRIEF DESCRIPTION OF THE DRAWINGS

In the drawings:

FIG. 1 schematically depicts principle of the Geophysical Tomographical method for a layered system with an elastic wave velocities gradient including heterogeneous media.

FIG. 2 schematically depicts the division of one vertical slice of the 3D observed volume of the object into a 2D array of elementary cell arranged in columns and rows and shows the locations of the transducers.

FIG. 3 schematically depicts a layered observed object including heterogeneous media with elastic wave velocity gradient, and the elastic wave propagations in the layered object.

FIG. 4 schematically depicts some details of a two layers subject, demonstrating that a composite, multi-layered object may be expressed as an equivalent two layers object.

FIG 5 demonstrates the direct and the reciprocal routes of propagating elastic wave for adjacent locations in the vertical slice according to the applied algorithm according to the invention.

FIG. 6 demonstrates some details of elementary cell selection.

FIG. 7 a - 7d demonstrate experimental images bone and surrounded bone tissues. The images in Fig. 7a-7b are obtained by the differential approach applying, the images Fig. 7c and 7d by the integral approach. The obtained images demonstrate insensitivity of the integral approach.

FIG. 8 schematically depicts a succession of measurements in the direct direction. FIG. 9 schematically depicts a succession of measurements in the reciprocal direction.

FIG. 10 schematically depicts a functional block diagram of the ultrasonic tomography device according to a preferred embodiment of the current invention.

FIG. 11 illustrates the experimental ultrasonic device with scanning transducers movement

Table 1 depicts obtained data and its treatment according to the algorithm of the current invention

Table 2 depicts the sequence of the calculations and calculated values derived from the measured data for specimen with predominate soft tissue.

Table 3 depicts the calculated longitudinal wave velocity values for specimen characterized by predominate bone tissue.

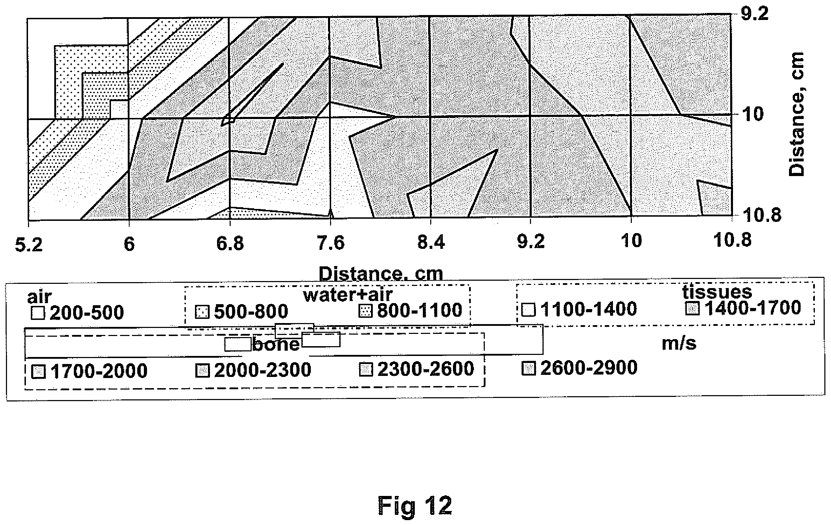

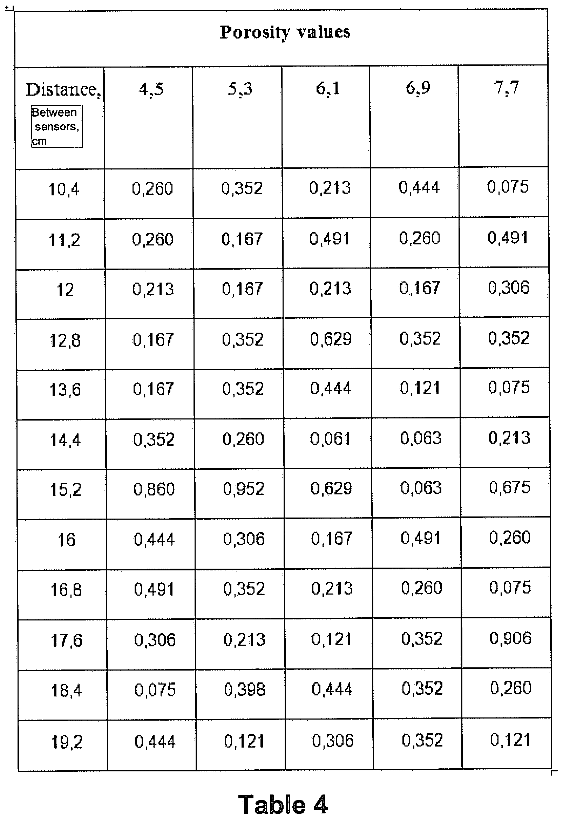

FIG. 12 shows experimental results for longitudinal wave velocity values distribution in the map of the slice of pig's leg. The images where pixel-by-pixel synthesized in Microsoft Excel®.

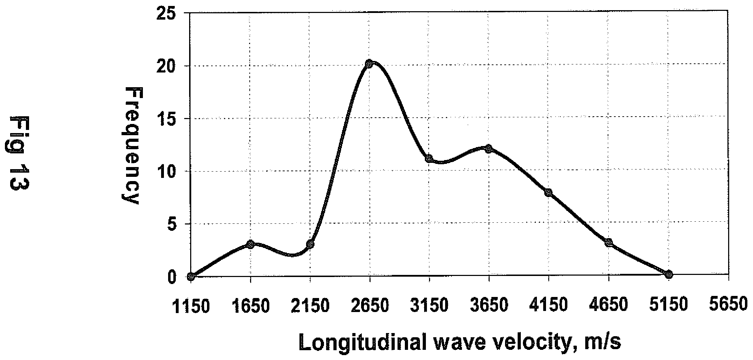

FIG. 13 shows a histogram of experimentally measured longitudinal wave velocity for plurality elementary cells providing longitudinal wave velocity in bone matrix evaluation.

Table 4 depicts the calculated porosity values for a specimen characterized by predominated bone tissue.

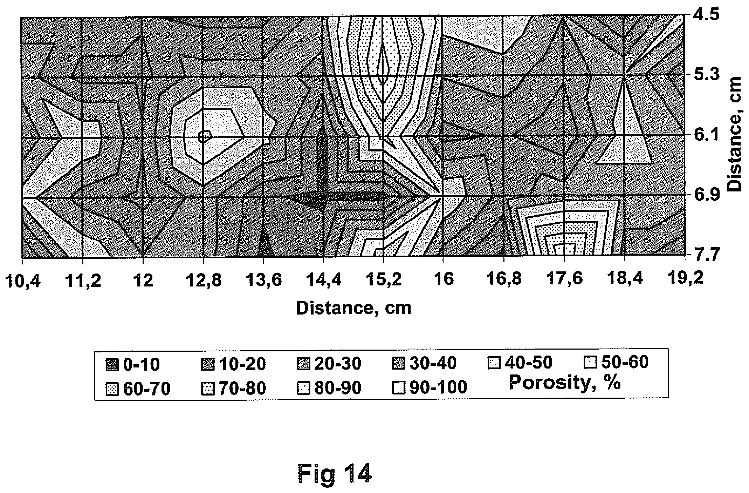

FIG. 14 shows experimental results for porosity value distribution in the slice of pig leg. The image was pixel-by-pixel synthesized in Microsoft Excel®.

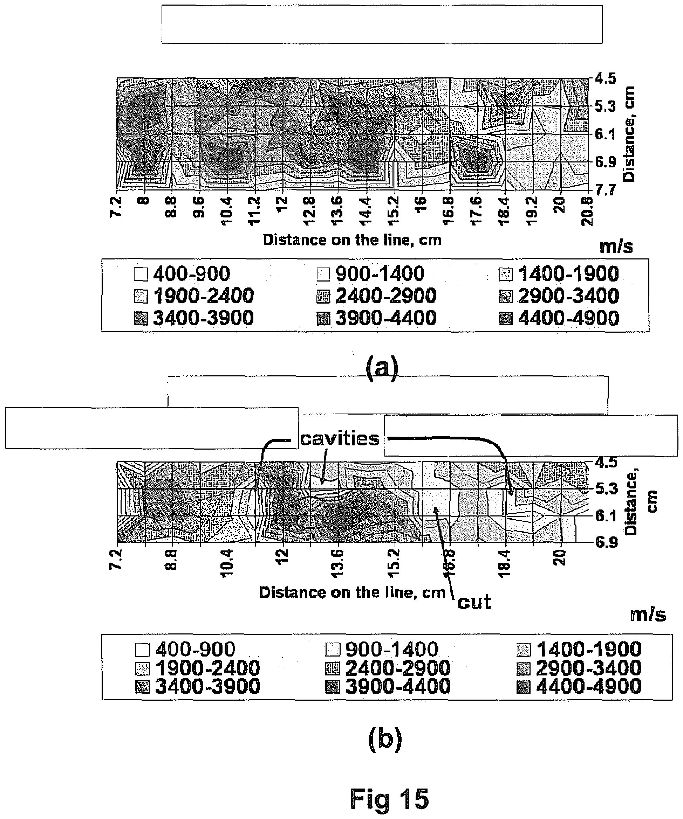

FIG. 15 illustrates experimental results demonstrating the possibilities of the proposed technology by of comparison images obtained for pig's leg before and after mechanical damages.

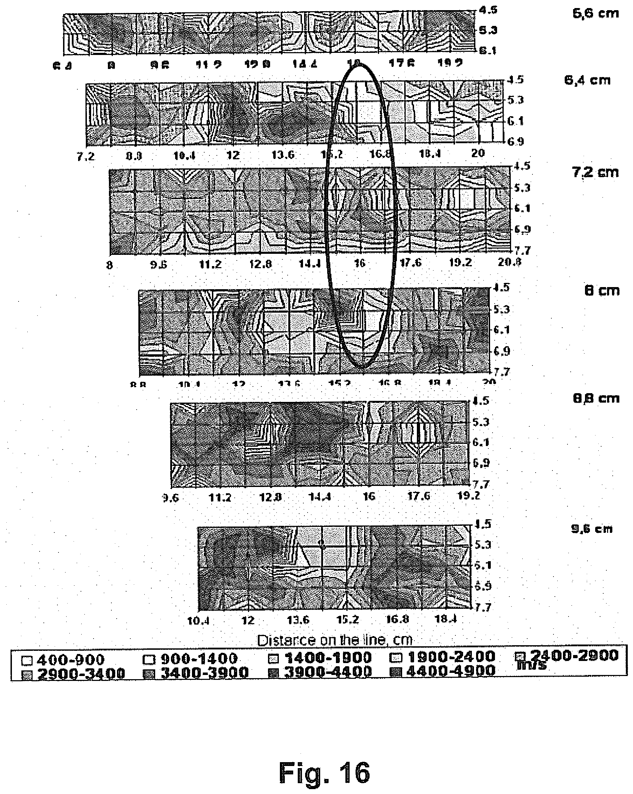

FIG. 16 illustrates experimental results demonstrating the possibilities of the proposed technology 3D bone quantitative imaging by virtue of 2D section repeating by the third axis with constant step b.

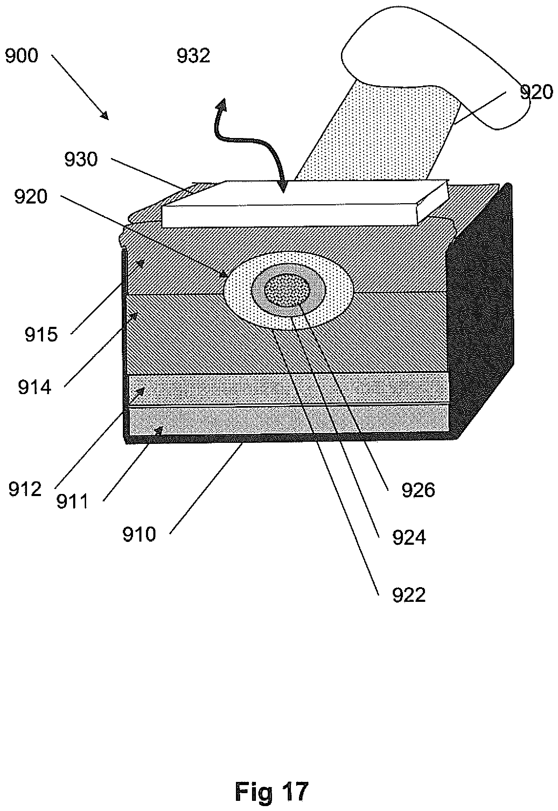

FIG. 17 schematically depicts an apparatus for inspection of organ such as a limb.

DESCRIPTION OF THE PREFERRED EMBODIMENTS

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

In discussion of the various figures described herein below, like numbers refer to like parts.

The drawings are generally not to scale. For clarity, non-essential elements were omitted from some of the drawings.

As used herein, an element or step recited in the singular and proceeded with the word "a" or "an" should be understood as not excluding plural elements or steps, unless such exclusion is explicitly recited.

The invention proposes novel approach to data measurements and treatments providing possibility for inspecting real heterogeneous and anisotropic substances by way of providing information about longitudinal wave velocity and porosity values for each elementary volume (elementary cell) from which the inspected object is composed.

. The known types of the ultrasonic wave propagations in an inspected object are described in the art. The method of refracted waves, the Pulse-Echo method, and time-of-flight diffraction TOFD method provide oscillations that reflect or refract on the boundaries between media with different acoustic impedance. They provide unilateral inspections of an inspected object and data about reflecting or refracting ultrasonic waves on boundary layer within the object.

The direct transmission method provides passage ultrasonic oscillations, so that a transmitter and a receiver are disposed from two sides of an inspected object.

The known geophysical tomographical method provides non-linear propagation of ultrasound waves. The aforesaid method is applicable for inspecting heterogeneous objects containing in layered systems that characterized by acoustic impedance gradient. The method provides possibility of the unilateral inspections of heterogeneous media.

But the known ultrasonic methods do not provide the required sensitivity in case of heterogeneous object such as bones. They demonstrate an integral approach based on averaging the obtained parameters such as amplitude or travel time. It does not take into account a situation when an inspected parameter is increased in one part of an inspected heterogeneous object and at the time is reduced in it's another part.

The invention proposes to apply the method of non-linear wave propagation for inspection of heterogeneous media and also proposes differential approach to data measuring and treatment. It provides unilateral object volume inspections. A proposed differential approach to data measuring and treatment provides sufficient sensitivity for the inspections of the heterogeneous materials. The provided by differential approach data corresponding to each element of the plurality of the object elementary cells are synthesized into 2D and 3D quantitative-images mapping according to a tomographical pixel-by-pixel approach..

The ultrasonic tomographical method based on simultaneously measuring and imaging according to a preferred embodiment of the present invention comprises a few principal approaches or aspects.

The first, it is the physical principle of elastic wavers distributions in the layered system with an acoustic impedance gradient including heterogeneous media. Parts of the human body to be inspected are considered as a layered system characterized by acoustic impedance gradient.

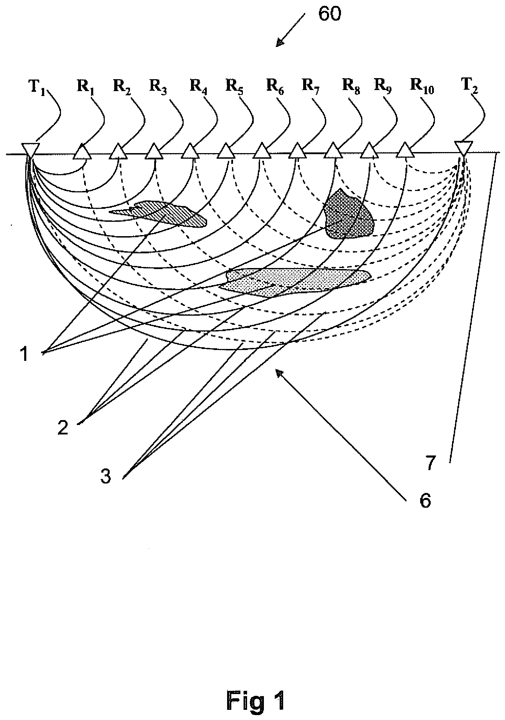

Figure 1 schematically depicts principle of the Geophysical Tornographical method for a layered system consisting of heterogeneous media with an elastic wave velocities gradient and containing heterogeneous objects.

The effect of the heterogeneous media was taken in account in the geophysical Tomographical method. Fig. 1 depicts the principle of the geophysical Tomographical method. The basis of this approach is the elastic waves non-linear travel (refraction) in layered systems associated with heterogeneous media (depicted as inclusions 1 in an inspected object 6), which have an elastic wave velocities gradient. Inspection system 60 comprises sensors $R_1$ to $R_{10}$ arranged along a linear profile on the surface 7 of object 6. The transducers $T_1$ and $T_2$ are situated at the opposite ends of the profile. Transducers $T_1$ and $T_2$ are used for transmitting sound wave as well as receiving sound waves.

Curved routes of waves propagating from Transducer $T_1$ to the sensors R1-10 and Transducer T2 (direct routes 2) are depicted in solid lines. Reciprocal routs 3, from transducer T2 toward receivers $R_{10}$ to $R_1$ and transducer $T_1$ are depicted in dashed lines.

The method is described, for example, in the work "Ore seismic" by N.A.Karaev and Ci.Ya. Rabinovich. -Moscow:

Geoinfonnmark Closed Joint- Stock Company, 2000. -366p, ill, and by L.A.Pevzner, V.L.Pokidov, V.A.Tsimer in the work "The seismic investigation of complex medium". Alma-Ata. Science, 1984. However this method is not used for the medical purposes.

The fact of non-linear beams routes is known from literature and is similar to propagation light beams in heterogeneous media system which characterized, for example, by temperature gradient and is known as "a mirage". The phenomenon is expressed by the fundamental Fermat principle. Similarly to light beams, elastic waves change its linear routes and reverse the route that offers the fastest time of travel. The geophysical method utilizes angle transducers aimed at right angle (90 degrees) to the surface of the observed object.

Similarly to the geophysical method the invention considers the inspected object as local hetrogeneous region and employs ultrasonic wave with length that is commensurable with dimension of the local heterogeneous region.

The TOFD technique is applied for providing different approaches to upper layer and to lower located layers of used layered system and provides possibility for homogeneous media inspections. The invention uses similarly to the TOFD method emitting and receiving of ultrasonic oscillations at an angle, but it is a constant angle in distinction from TOFD method. The geophysical method is not applied in medicine as it can not to provide the needed resolution and sensitivity for medical investigations. It applies the known integral approach for data measuring and treatment.

The second approach, it is the Ultrasonic Computer Tomography of elastic wave velocity (porosity) accounting the influence of heterogeneity and anisotropy of investigated heterogeneous media and providing images mapping.

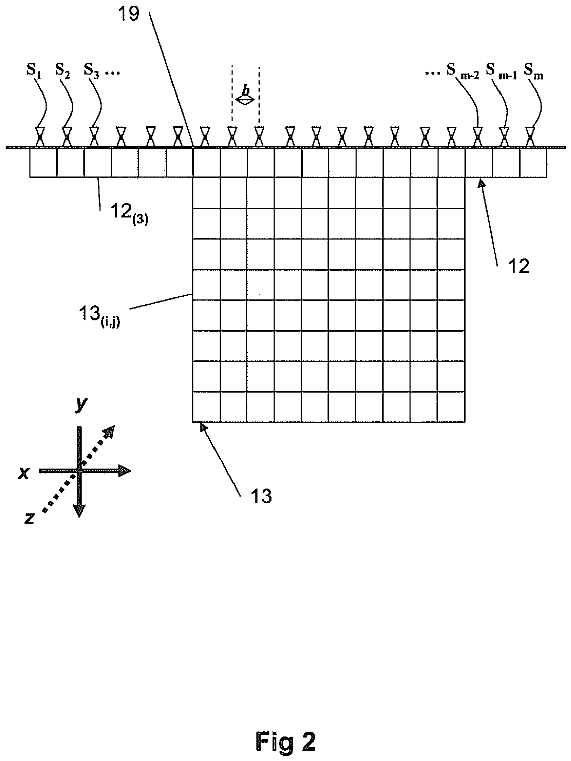

FIG. 2 schematically depicts the division of one vertical slice of the 3D observed volume of the subject into a 2D array of elementary pixel arranged in columns and rows and shows the locations of the transducers.

For simplicity, one vertical slice of the observed three-dimensional (3D) object is depicted. An inspected volume (13) is considered as a plurality of elementary cells (cubes) as shown in the FIG. 2. A distance between transducers ($S_1$, $S_2$, $S_3$.... $S_{m-1}$, $S_m$) is constant and is equal to $b$. The value $b$ defines a distance between profiles (lines of grid) that in turn separate the elementary cell. The elementary cell of the first layer of the inspected layered system is indicated by a numeral 12. The picture presents plurality of elementary cells arranged into column and rows. Itis to be understood that plurality of such two-dimensional (2D) slices may be combined to create representation of the full 3D object. For example, plurality of adjoining parallel slices may be used to create a 3D representation of the observed volume. The pixel may be chosen as cubical having an elementary volume of $b*b*b = b^3$, but may have distorted sizes in the third dimensions by the axis beam penetration, that may be account. In the depicted example, the vertical slice is in the $X$-$Y$ plane, while adjoining slices may be spaced in the $Z$ direction with a constant step which is equal to b. Such method of 3D bone imaging by means of integrating a plurality of 20 images will be described further with reference to figure 16.

Any number of slices, sensors and any number of columns and rows may be chosen within the general aspects of the current invention.

Sensors $S_1$ to $S_m$ are preferably equally spaced, with spacing $b$ along a line on the surface 19 of the observed object 13. It is desirable that the greater acoustic beams penetration into an inspected body and a condition of heterogeneous substances for acoustic inspections must be kept. The condition of heterogeneous substances for inspection by means of acoustic methods is expressed through ratio between a length of longitudinal ultrasonic wave $\lambda_{wave}$ and a dimension $d$ of a considered material grain such that $\lambda_{wave} \leq d$..

According to the conditions, the preferred frequency for bone media inspections lies in the range of low frequencies. For example it is 50 kHz or 100 kHz. It satisfies the two conditions.

The method allows "low frequency tomography" of large objects. Additionally, high frequency ultrasonic waves are reflected and deflected by the bones and cavities filled with gas. Thus the bones and structures cannot be imaged. In contrast, the waves having low frequencies used according to the invention are refracted by the bones and thus may be imaged.

Incontrast to geophysical systems as present in figure 1, wherein only two transmitters were used, and wherein said two transmitters were located at the two opposing ends of the sensors[1] profile, the system according to the current invention uses transducers that are capable of emitting and receiving the ultrasonic waves.

Preferably, the transceivers are constructed such that the emitted and received acoustic waves are directed at an angle to the surface. For example, a wedge provides required angulations when the transducers emit or receive the ultrasonic wave. Preferably, the waves are emitted and received at an angle more than 30 degrees to the surface. The angle value influences on beam penetrations into the investigated object. Preferably, the emitted wave is directed towards the sensor used as receiver at more than 30 degrees, and the receiver is similarly angled.

The required slanting of the system sensors can be achieved by rotating the wedge, by positioning two oppositely angled transceivers at a location, by mechanically tilting the transceiver or by electronically changing the effective line of sight of a piezoelectric phased array.

For clarity, the first row 12 of elementary pixels in the slice is differently marked. For example pixel $12_{(3)}$ is the pixel directly underneath sensor $S_3$. The pixels in the rest of the slice are marked according to their row and column, for example $13_{(lj)}$

Preferably, index matchingjell is used to efficiently couple acoustic energy into and out of the object.

Preferably, acoustic properties of the layer located adjacently with respect to the sensors should be known. This could be achieved by realizing that the first layer of the system can be an additional plate or/and pillow having known acoustic properties and that in the human body it can be the skin sometimes with known properties. The value may be calculated also, but one needs to take into account that the length of routes of elastic wave in the pixel of the first layer differs from the length of routes of elastic waves propagating through cells of other layers. Additionally or alternatively, a "pillow" filled with the jell may be placed between the object and the sensor array. Alternatively, a "pillow" made of a solid material that has properties of water can be placed between the object and the sensor array. Alternatively, the object may be immersed in a container with liquid with known acoustic properties. Immersion and jell-pillow may be used for interfacing a generally curved surface of a human body and the plate surface carrying the sensor array.

The human body is considered as a layered system comprising skin, fat, periosteum, cartilages, muscles and bone. The human body is considered as a layered system containing heterogeneous media and is characterized by an acoustic impedance gradient.

The layered system may be provided by attaching suitably configured artificial additional layers or parts. By virtue of this provision the ultrasonic waves propagate non-linearly in such a complex layered system.

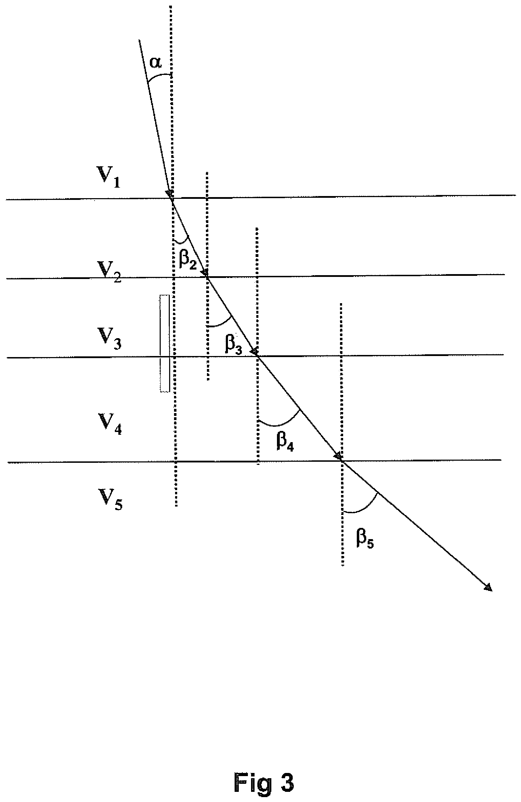

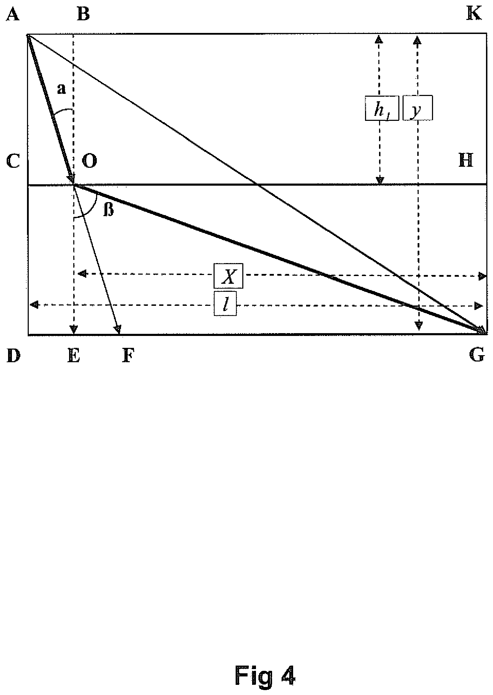

The fourth aspect of the invention is the differential approach to data measurements and treatment providing possibility for tomographical mapping of data corresponding to plurality of elementary cells. This approach provides the measurements sufficient sensitivity. The following figures 3, 4 and 5 explain the differential approach for heterogeneous media inspections. FIG. 3 and FIG.4 ullustrate the non-linear traveling of the ultrasonic beams in such layered system under inspection.

FIG. 3 schematically presents an inspected layered system containing heterogeneous media characterized by elastic wave velocity (acoustic impedance) gradient, and the elastic wave propagations in the layered system.

The layered system characterized by an acoustic impedance gradient including heterogeneous media. The possible directions or beam travels associated with waves propagating in the system are shown in the FIG. 4. The beam travels correspond to the Snell's reflection law:

$$Sin\ a\ /\sin \beta\ = V_A/V_B,$$

Where sin a is Sine of the incident angle on the boundary between media A and B with different acoustic impedance, sin $\beta$ is the refracted angle in the second media, $V_A$ and $V_B$ are longitudinal wave velocities in the first and the second media for the case when the system consists from two layers.

An observed subject is composed from a few layers including heterogeneous media and has an elastic wave velocity gradient. In the example of figure 3, five layers are seen with gradient velocities. The condition may be expressed as:

$$V_1 < V_2\ < V_3 < V_4\ < V_5$$

According to the Snell's refraction law for the considered boundary between the media 1 and 2 of the considered object depicted in figure 3, the propagating wave will obey the following condition:

$$\frac{\sin \alpha}{\sin \beta_2} = \frac{V_1}{V_2} \qquad (1)$$

Where sina is sine of the incident angle on the boundary between media 1 and 2 with different acoustic impedance, sinβ2 is the angle of the refracted beam in the second medium, $V_1$ and $V_2$ are the longitudinal wave velocities in the first and the second media.

From the equation 1 we can write down:

$$\sin \beta_2 = \sin \alpha \times \frac{V_2}{V_1} \qquad (2)$$

Similarly, for the boundary 2 which is the boundary between media 2 and 3:

$$\frac{\sin \beta_2}{\sin \beta_3} = \frac{V_2}{V_3}$$

Where sin ß₂ is sine of the incident angle on the boundary between media 2 and 3 with different acoustic impedance, sin ß₃ is the refracted angle in the third medium, $V_2$ and $V_3$ are the longitudinal wave velocities in the second and the third media.

Sin ß₃ may be expressed as:

$$\sin \beta_3 = \sin \beta_2 \times \frac{V_3}{V_2}$$

and thus as:

$$\sin \beta_3 = \sin \alpha \times \frac{V_3}{V_1}$$

For the boundary 3, the boundary between the media 3 and 4 we can write:

$$\frac{\sin \beta_3}{\sin \beta_4} = \frac{V_3}{V_4}$$

Where sin ß₃ is sine of the incident angle on the boundary between the media 3 and 4 with different acoustic impedance, sin ß₄ is the refracted angle in the fourth medium, $V_3$ and $V_4$ are the longitudinal wave velocities in the third and the fourth media;.

Sin ß₄ may be expressed as:

$$\sin \beta_4 = \sin \alpha \times \frac{V_4}{V_1},$$

And by analogy for the boundary n-1, which is the boundary between the media n-1 and n the relations may be written as:

$$\frac{\sin \beta_{n-1}}{\sin \beta_n} = \frac{V_{n-1}}{V_n}$$

And thus as:

$$\sin \beta_n = \sin \alpha \times \frac{V_n}{V_1},$$

Hence, a multi-layered object with an elastic wave velocities gradient comprising layers maybe expressed similarly to a two-layered object.

FIG. 4 schematically depicts some details of a two layered object, demonstrating that a composite, multi-layered subject may be expressed as an equivalent to a two layers subject.

The region AKCH represents the first layer and CHDG as the *n*-th layer of the layered object.

The longitudinal wave velocities in the layers are depicted by $V_1$ and $V_n$ respectively.

The distance AK = *l*, is half of a distance between transmitter and receiver for each from a sensor's arrangement. $h_1$ is the thickness of the first layer, sin a is the sine of the incident angle in the considered boundary and the sin ß$_n$ is the sine of the refracted angle in the medium n.

According to Snell's relationship for the layers:

$$\frac{\sin \alpha}{\sin \beta_n} = \frac{V_1}{V_n}$$

The expression $\sin \beta_n \times V_1 = \sin \alpha \times V_n$ describes an average velocity value for oscillations travel from point A to point G.

According to Fermat's principle, ultrasonic oscillation travels along the fastest route.

Defining the distances: EG as *x*, and AD as y, and

$S_{AG}$ as the shortest distance from point A to point G; $S_{AG}$ may be expressed as:

$$S_{AG} = \sqrt{\ell^2 + y^2}$$

And thus the shortest time $\tau_{short}$ may be written as: $S_{AG}/ (\sin a * V_n)$ or as

$$\tau_{short} = \frac{\sqrt{\ell^2 + y^2}}{\sin \alpha \times V_n}$$

$$\sin\alpha = \frac{\ell - x}{\sqrt{(\ell - x)^2 + h_1^2}}; \sin\beta_n = \frac{x}{\sqrt{x^2 + (y - h_1)^2}}; \frac{\sin\alpha}{\sin\beta_n} = \frac{(\ell - x) \times \sqrt{x^2 + (y - h_1)^2}}{x \times \sqrt{(\ell - x)^2 + h_1^2}} = \frac{V_1}{V_n}$$

$$\tau_{short} = \frac{\sqrt{\ell^2 + y^2} \times \sqrt{(\ell - x)^2 + h_1^2}}{(\ell - x) \times V_n}$$

Similarly, the shortest time may be written and as the corresponding distances divided by the corresponding velocity values: $S_{AO}/V_l + S_{OG}/V_n$ or as:

$$\tau_{short} = \frac{\sqrt{h_1^2 + (\ell - x)^2}}{V_1} + \frac{\sqrt{x^2 + (y - h_1)^2}}{V_n}$$

We can write the equation from the shortest time:

$$\frac{\sqrt{\ell^2 + y^2} \times \sqrt{(\ell - x)^2 + h_1^2}}{(\ell - x) \times V_n} = \frac{\sqrt{h_1^2 + (\ell - x)^2}}{V_1} + \frac{\sqrt{x^2 + (y - h_1)^2}}{V_n}$$

We can then divide the both part of the equation by $\sqrt{(\ell-x)^2+h_1}$ to get:

$$\frac{\sqrt{\ell^2+y^2}}{(\ell-x)\times V_n}=\frac{1}{V_1}+\frac{\sqrt{x^2+(y-h_1)^2}}{V_n\times\sqrt{(\ell-x)^2+h_1}}$$

The equation may be written for direct and reverse directions as:

$$\sqrt{\ell^2+y^2}=\frac{(\ell-x)V_n}{V_1}+\frac{V_1\times x}{V_n}$$

$$y^2=\frac{(\ell-x)^2V_n^{\;2}}{V_1^{\;2}}+2\ell x-x^2-x^2-\ell^2+\frac{V_1^2 x^2}{V_n^{\;2}}\;;$$

$$y^2=(\ell-x)^2\left|\frac{V_n^{\;2}}{V_1^{\;2}}-1\right|+x^2\left|\frac{V_1^{\;2}}{V_n^{\;2}}-1\right|\;;$$

Or as

$$y^2=(\ell-x)^2\left|\frac{\sin^2\beta_n}{\sin^2\alpha}-1\right|+x^2\left|\frac{\sin^2\alpha}{\sin^2\beta_n}-1\right|$$

The dependence is described by the equation of the second order. It is a non-linear function.

The maximal depth of a beam penetration for refracted wave is determined by the gradient velocity values $(V_n/V_l)$ of media and by the distance between a transmitter and a receiver *2l*.

Hence, a system for the Ultrasonic Tomographical measurements may be based on the changing of distance between transducers and receivers which are situated at one surface of an inspected object.

We can assert that significant number of non-linear routes exists which are distributed in the same layered system including heterogeneous media characterized by an elastic wave velocity (acoustic impedance) gradient.

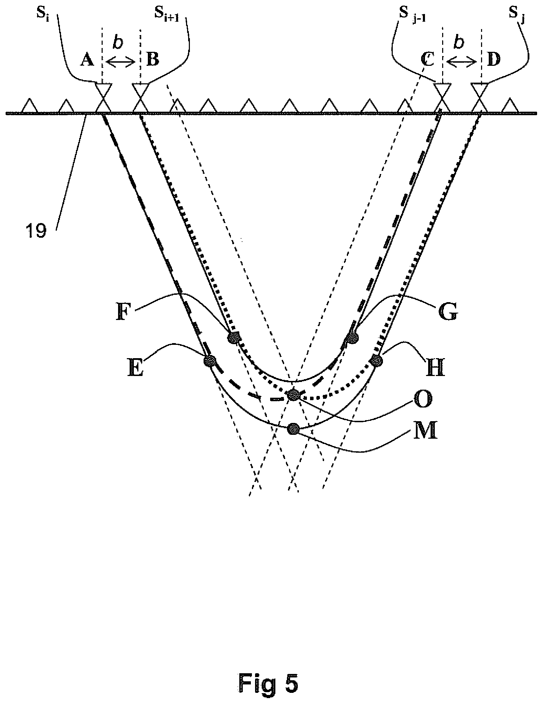

FIG 5 demonstrates the direct and the reciprocal routes corresponding to each combination of elastic wave propagating from a pair of adjacent transmitting transducers to a pair of adjacent receiving transducers and select elementary volume (cell) in the vertical slice according to the applied algorithm according to the invention.

The invention utilizes the algorithm, which provides differential measurement and treatment of obtained data. It shows the measurement system and the propagation elastic wave according to the invention.

The sensors $S_i$, $S_{i+l}$ and sensors $S_{j-l}$, $S_j$ are situated on the surface 19 in locations A, B, C, and D respectively.

Transmitters (or transducers used as transmitters) located in points A and B emit ultrasonic oscillations at an angle to an investigated object surface 19. The oscillations are received by receivers (or transducers used as receivers) that situated at locations C and D which are angulated accordingly.

Four routes may be plotted in the direct direction: AC, AD, BC, and BD.

Similarly, we obtain the four routes in the reciprocal direction: CA, DA, CB, and DB, where the transmitters are situated in the points C and D and receivers in the points A and B.

The FIG. 5 explains the proposed algorithm of the differential approach, which is based on adding up and subtraction of the longitudinal wave travel times for the certain combination of the direct and reciprocal routes associated with adjacent locations. If we add up and subtract the average values of the longitudinal wave travel times that are needed for propagating of longitudinal wave along the routes in direct and reciprocal directions we can obtain the travel times difference for a considered cell relative to the adjacently located cell.

It should be noted that the angulations of the emitted and received beams such as depicted by the lines AE, BF and GC, HD is preferably 45 degree to surface 19, however, smaller or larger angles are also possible.

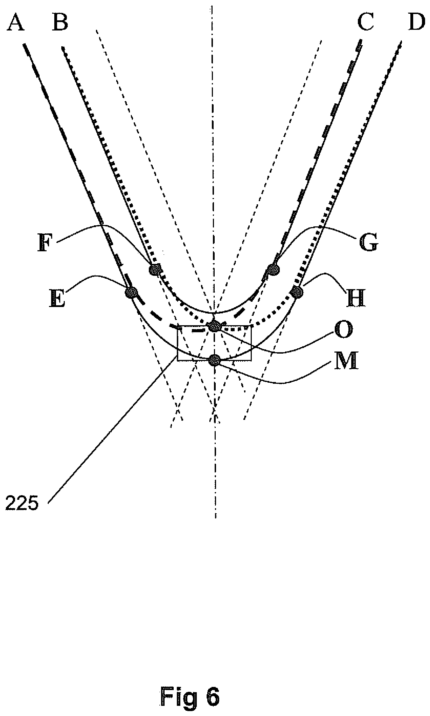

FIG. 6 demonstrates some details of elementary volume selection. Figure 6 present the proposed algorithm of evaluating of the changes travel time value $\Delta t_m$ in each subsequent cell relative to previous cell of the column within the inspected object. The obtained difference in travel times $\Delta t_m$ relates to the considered cell 225 relative to previous cell and corresponds to the transducers arrangement with step $b$ on the surface 19 (see Fig.5) along central line joining the points O and M as depicted in figure 6.

The Prior art methods, including the geophysical method, consider only two signals for selection of the part of an inspected object. In contrast, the inventive algorithm employs a combination of eight signals for revealing properties of a specific elementary volume in the observed object.

The figure 6 explains the proposed algorithm. Consider the difference of obtained average between direct and reciprocal directions times for signal traveling between points A - D and points B - C. The obtained difference $t_{AD} - t_{BC}$ characterizes the changes in the selected half arc seen in the picture. Consider the difference of obtained average times for signal traveling between points A - C and points B - C. The obtained difference $t_{AC} - t_{BC}$ characterizes another selected part of the considered arc. Consider the difference of obtained average times for signal between points B - D and points C - B. The obtained difference $t_{BD} - t_{CB}$ characterizes the additional selected part of the considered arc.

The needed elementary volume may be obtained by the way of subtraction of the signals that characterize the selected parts of the half of the ring, which are selected in the pictures depicted in the FIG. 5 and 6.

$$\Delta t_m = (t_{AD} - t_{BC}) - (t_{AC} - t_{BC}) - (t_{BD} - t_{CB}) = t_{AD} + t_{BC} - t_{AC} - t_{BD}$$

The needed part of an inspected object is the selected part in FIG. 6.

The considered difference $\Delta t_m$ is the difference between smallest time ultrasonic oscillations travels along the curvilinear routes in the considered cell ($T_{EM} + T_{MH}$) and in the above placed cell ($T_{EO} + T_{HO}$).

$$\Delta t_m = T_{AE} + T_{EM} + T_{MH} + T_{HD} + T_{BF} + T_{FO} + T_{OG} + T_{GC} - T_{AE} - T_{EO} - T_{OG} - T_{GC} - T_{DH} - T_{HO} - T_{OF} - T_{FB}$$

$$\Delta t_m = T_{EM} + T_{MH} - (T_{EO} + T_{HO})$$

The calculations of a longitudinal wave average velocity value for each cell are carried out by using values of longitudinal wave travel times in each cell and of a length of travel of longitudinal wave in a cell.

We apply the approach by substitution of the considered composed multi-layered object with an equivalent object constituting of two layers. The observed layer is determined by the considered elementary volume (cell), in which longitudinal wave propagations follow non-linear routes. We define the travel way in an elementary cell of an examined composted system by an estimated value, calculated as half of length of a circle with radius $b$ that is $\pi b$. We assume that the length of travel in the single first known layer is $2b/sin\alpha$ ($\alpha$- is the incident angle to said layered system) or $2\sqrt{2} \times b$ for the incident angle of the first layer 45°, were $b$ is the distancebetween transducers.

The proposed invention may be used for inspection of homogeneous substances also, but the length of travel of a single in all elementary cells will be calculated according to $2b/sin\alpha$ or for incidence angle 45° the equation would be$2\sqrt{2} \times b$.

The invention proposes differential way for measurements and treatments of an obtained data that provides evaluation each elementary volume of an inspected object.

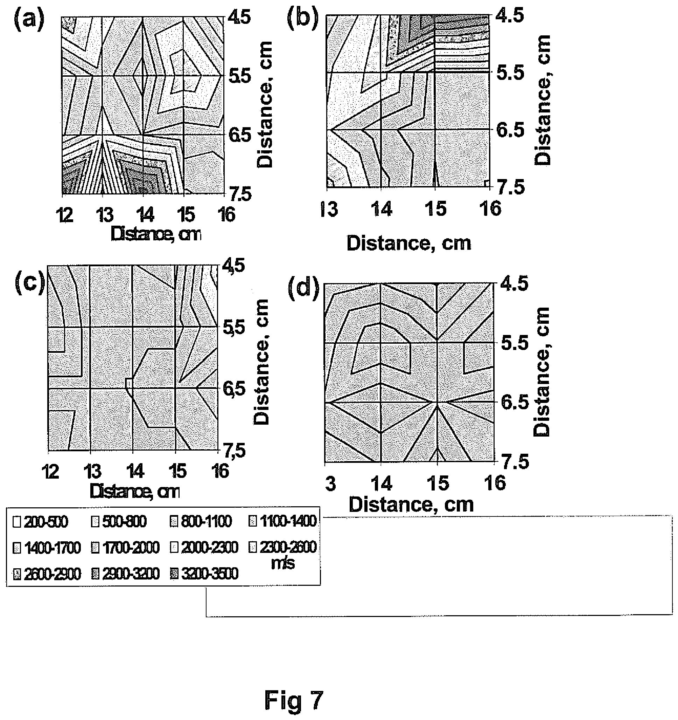

The FIG. 7a - 7d present experimental images of the object including a bone and surrounding soft tissues. The images (Figure 7c and 7d) were obtained by means of the integral approach and they demonstrate insensitivity of the aforesaid approach to local changes of the longitudinal wave velocity within the inspected object and not reveal the bone and the boundary between bone and soft tissue (figures 7c and 7d) as it shown in the Figures - 7a and -7b for the differential approach.

Majority known methods in different fields use integral approach that does not provide possibility for inspecting heterogeneous materials (as bone) with sufficient sensitivity. The obtained experimental results demonstrate the improved sensitivity of the proposed differential approach.

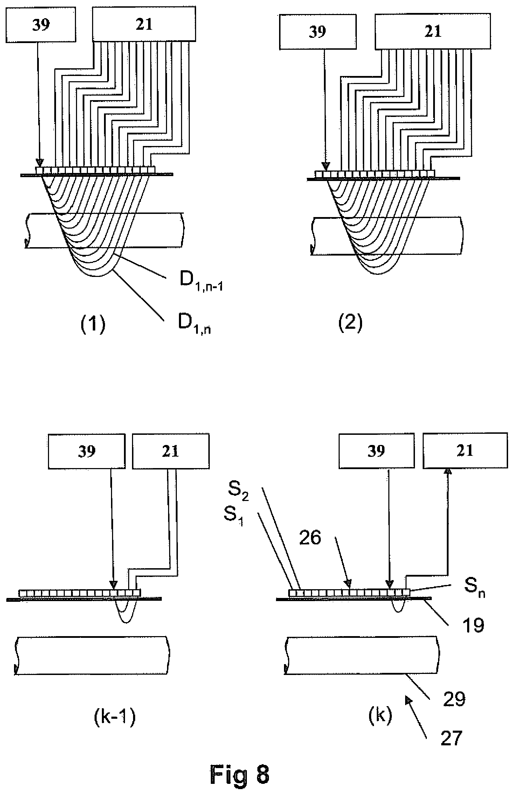

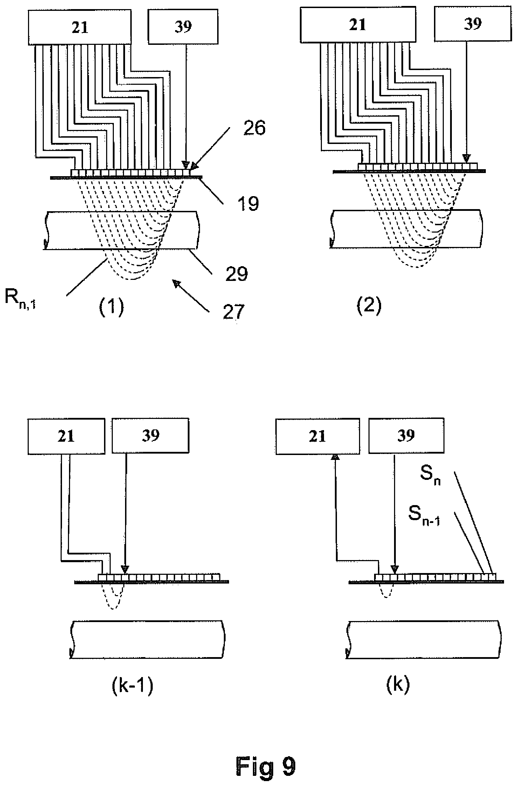

Figures 8 and 9 schematically depict the method of data acquisition according to the current invention: FIG. 8 schematically depicts a succession of measurements in the direct direction, while FIG. 9 schematically depicts a succession of measurements in the reciprocal direction. For simplicity, the 1D array of sensors is shown. Extension of the method to the 2D sensor array is done by activation each row of the sensors at a time.

For simplicity, not all parts in the drawing were labeled.

According to a preferred embodiment of the invention, electronic signal is transmitted from the scanning controller 39 to one of the transducers $S_i$ in the row of transducers 26 which is in contact with the surface 19 of the observed object 27 having internal structure 29.

Figure 8(1) depicts the situation in which the first sensor $S_1$ in the row is used as a transmitter. The first sensor $S_1$ is angled to the right by using a wedge or other angulation's means.

Paths of acoustic beams from sensor $S_1$ arriving to other sensors in the line are seen. These sensors are used as receivers and are angulated to the left along the marked paths. Direct paths are marked as $D_{ij}$ wherein i is the index of the transmitter and j is the index of the receiver. Electronic signals from the receivers are transmitted to the measuring time unit 21 which detects and measure the arrival time of acoustic signal to the sensor, thus determine the time of flight from sensor i to sensor j along the direct path $D_{ij}$, which is the time $t_{ij}$. For simplicity, only $D_{l,n}$ and $D_{1,n-1}$ are marked in drawing 8(10). Measuring time unit 21 may process one signal from one sensor at a time or may be a processing unit capable of parallel processing plurality of signals at a time..

In figure 8(2), the second sensor $S_2$ in the line is used as a transmitter and remaining sensors as receivers. The sensor $S_2$ is angled to the right by using a wedge or other angulation means. Paths of acoustic beams from sensor $S_2$ arriving are seen. The beams arrive to other sensors in the line which are used as receivers and are angulated to the left along the marked paths. Optionally, sensor $S_1$ is idle at that time.

The measurement process repeats until most of the sensors are used as transmitters as depicted in figures 8(k-1) and 8(k), wherein k is smaller than the number of sensors in the array n since generally the same sensor is not used as both transmitter and receiver at the same time, and in fact a minimal gap is equal to about 2-3 wave length of applied oscillations between transmitter and receiver (one sensor in the case depicted here) may be between the closest sensors used.

Reciprocal paths $R_{i,j}$ are similarly marked in dashed lines in figures 9(1), 9(2), 9(k-1) and 9(k).

For measuring reciprocal paths, the receivers are angulated to the right while the transmitters are angulated to the left.

It is understood that the sequence of measurements may optionally vary. It may be changed systematically or even randomly within the same row or among the rows and columns in a 2D array. However, it is preferred that all transmitter-receiver pairs in each line would be measured at least ones.

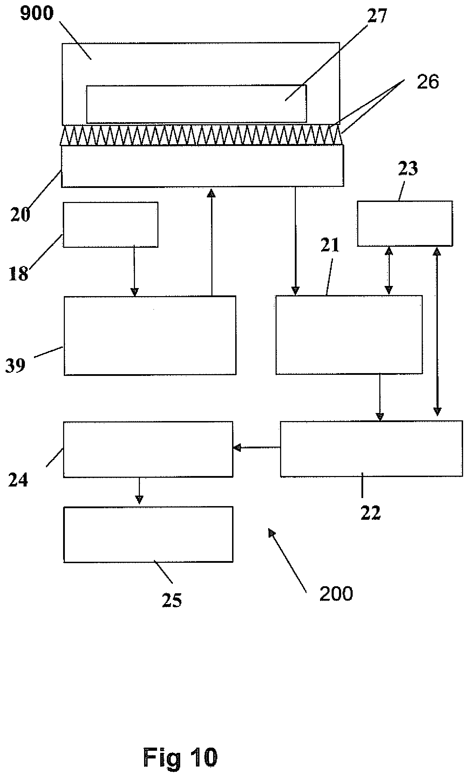

FIG. 10 schematically depicts a functional block diagram of the Ultrasonic Tomograph.

Ultrasonic Tomograph 200 is used to measure objects such as parts of a human patient 27 and obtain a map of material's and structural properties distribution in the observed object. Additionally, Tomograph 200 may detect bone weaknesses such as fractures and by this way estimate risk of bone fracture and reveal its location, etc.

Signal generator 18 produces electronic signal in the form of short pulses. The generated signal is directed by a scanner position controller 39 to one of the transducers 26 in the system set sensors 20 of the three dimensional ultrasonic system 900 including layered system. Scanner position controller 29 also controls the angulations of transducers 26. For example, each transducer 26 may comprise two transducers oppositely angulated. Alternatively, scanner position controller 39 may control electro-mechanical devices such as solenoids (not shown) within sensor set system 20 for changing angulations of transducers 26. Additionally, and optionally scanner position controller 39 may control an electro-mechanical device such as a stepper motor (not shown) within system set sensors 20 for scanning the array of transducers over the surface of observed object 27.

Signals from transducers 26, indicative of received ultrasonic signals are directed from three dimensional ultrasonic system 900 including system set sensors 20 to a measuring time unit 21. The unit 21 receives signals, amplify, filter and process them to measure shortest travel times along the direct and reciprocal paths. The measured travel time, in digital format is transferred to processor 22 which calculates the differential data according to the disclosed algorithm and stores the data in a storage device 23. Optionally the storage device can be a digital memory or a disk for storing the data produced by signal processor 21. Optionally, storage device 23 serves other digital units such as processor 22 and/or an image formation unit 24.

Image formation unit 24 uses the obtained data associated with plurality of elementary volumes for pixel-by-pixel images mapping distributions that of inspected bone property of an inspected bone and the obtained maps analysis. The results are displayed by a display 25. Optionally, image formation unit 24 gives interpretation of obtained images by revealing distinctive zones with physiological details of the inspected object.

It should be noted that processor 22 and image formation unit 24 may be in a form of a general purpose computer such as a PC or a laptop and that processing and image formation may be programmed in various suitable computer languages such as C++, Excel or MatLab or by other program.

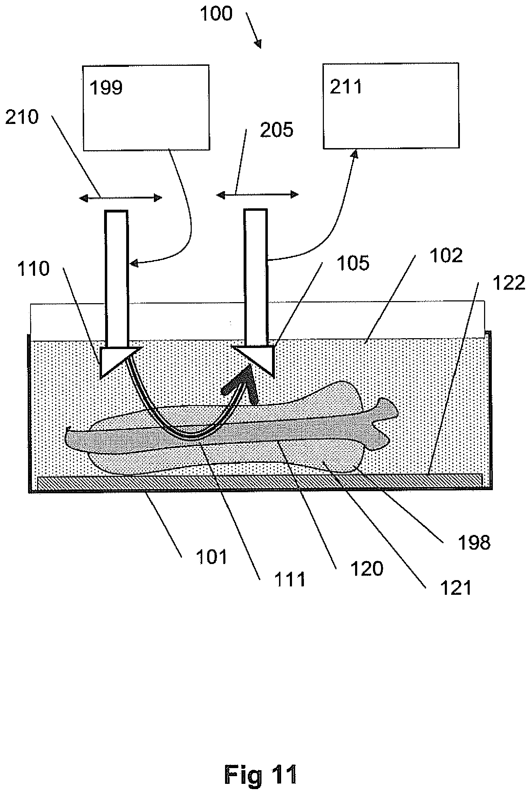

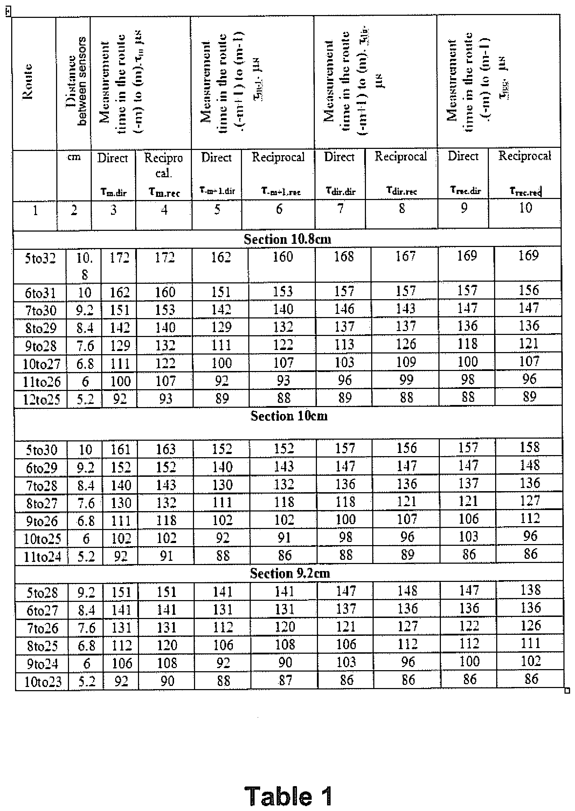

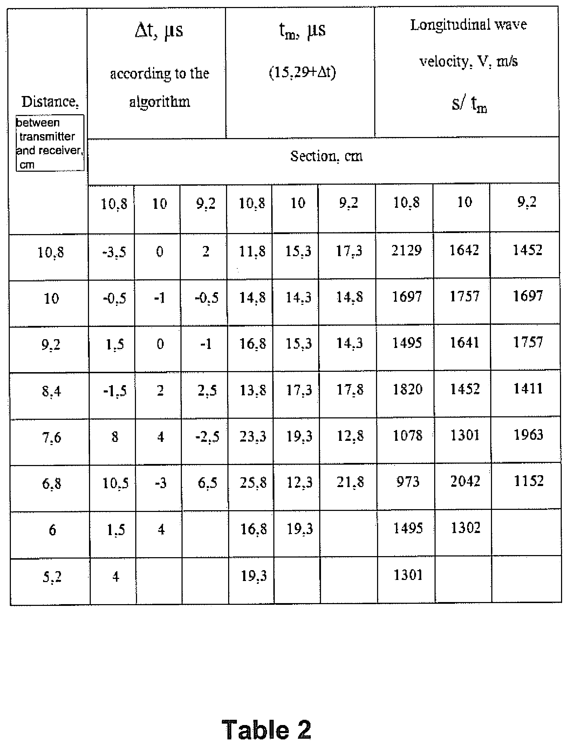

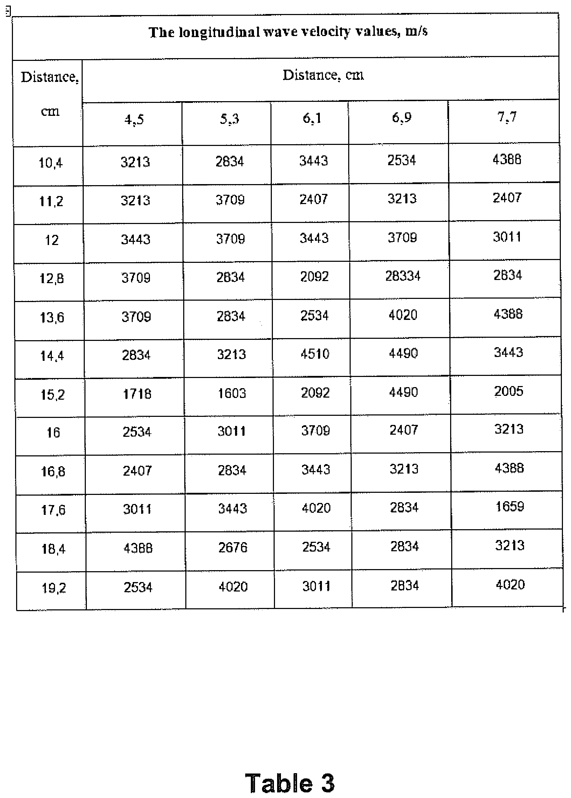

FIG. 11 illustrates the experimental ultrasonic system with scanning transducers movement which was used for the experimental demonstration and verification of the method.

Experimental system 100 uses a single transmitter 110 and a single receiver 105. Both transmitter 110 and receiver 105 are immersed in a liquid I02 contained by a container I01. An observed object 121 is immersed in the liquid and rests on a plate 122 (additional artificial part with high acoustic impedance) at the bottom of container 101. A pig's leg having a soft tissue 198 (the bone was wrapped with chicken meat for the needed inspected system creation) and a bone 120 was used in this experiment. Two pig legs samples are present in the current description..

In the experiment, the container was 40 cm long in the X direction, and 28.2 cm in its center were imaged. The container's depth was 12 cm in the Y direction. The container width was 17 cm in the Z direction.

Transmitter 110, which is angulated as depicted in the drawing, receives electronic signal from a pulse generator 199 and produces an ultrasonic beam 111. Beam 111 non-linearly travels within the complex layered volume: liquid 102 and the observed bone 120 soft tissue 121 and plate 122 and after passing it arrives at the receiver 105. A measuring unit 211 was used for extracting time of travel information.

Mechanical translators (schematically depicted by the double headed arrows 210 and 205 were used for translating transmitter 110 and receiver 105 respectively to the proper locations.

An old "concrete tester" made by C.S.I. (Holland) was used for this experiment. This device was not optimized for the purpose, yet it demonstrated the feasibility of the method according to the current invention.

Table 1 depicts obtained data and its treatment according to the algorithm of the current invention. Distance between sensors and between profiles was 8 mm. The treatment of the data was executed according to the algorithm proposed in the summary of the description. The following parameters were used: $\Delta\tau_m = \tau_m + \tau_{m-1} - \tau_{dir} - \tau_{rec}$ Where $\tau_m$ is the average value between longitudinal wave travel time for direct and reciprocal directions for one sensors arrangement (were m is a number of a sensor's location) from point m to point (-m); $\tau_{m-1}$ is an average value between longitudinal wave travel time for direct and reciprocal directions for sensors arrangement from point (m-1) to point (-m+1); $\tau_{dir}$ is an average value between longitudinal wave travel time for direct and reciprocal directions for sensors arrangement from point (-m) to point (m-1) and $\tau_{rec}$ is an average value between longitudinal wave travel time for direct and reciprocal direction for sensors arrangement from point m to point (-m+1). The sample was immersed in water and the velocity in water is 1480m/s (at 20°C).

The step of two-coordinate displacement of the transducers was 8mm. The length of the route which the wave travelled in a cell for the first layer is $2 \times \sqrt{2} \times 8 = 22{,}63mm$. The time of longitudinal wave travel in the first layer is thus computed to be 15.29μs.

$$t_1 = \frac{22{,}63 \times 10^{-3}}{1480} = 15{,}29 \ \mu s$$

The length of longitudinal wave travels in each cell is equal 2πb=3.14*8 mm, which was computed to be S=25.12mm.

The calculation procedure is presented in the table 2 for the specimen where soft tissues predominate.

Table 3 depicts the calculated longitudinal wave velocity values for another specimen where bone predominates.

The map of longitudinal wave velocity distributions in object was obtained from the obtained data using Microsoft "Excel" programming.

The fifth aspect is contouring distinctive details in said inspected object utilizing distinctive values longitudinal wave velocity for the details. Itispossible because each healthy organ has its own value longitudinal wave velocity and physic- mechanics properties estimations. For example, the approximate longitudinal wave velocity values in different human parts are: in soft tissue ~1540 M//S in bone ~ 1700 - 5000 M//S in fatty tissue ~ 1450 M/ /S in liver ~ 1550 M//S in blood ~ 1570M//S in muscle ~ 1580M/S. Changes in the velocities may indicate on diseased or an injured tissue such as fractured or osteoporotic bone. The approach is useful in the case bone inspections because for bone media the wide range of elastic wave velocity changes exists.

FIG. 12 demonstrates the distribution of the longitudinal wave velocity values in a section. The image provides revealing details in obtained images. The image was obtained as result of the inspections of the specimen of pig's leg, which included both soft tissues and bone media and where bone tissues predominates. The obtained image demonstrates the possibility to reveal anatomical details for diagnostics purposes. The velocity values relate for example to air within the range between the corresponding longitudinal wave velocity value from 360 up to 500 m/s, for water with air from 500 to 1480 m/s, for soft tissues 1400 -1600 m/sec and for bone tissues from 1700 up to 2900 m/s for the concrete specimen. The ranges of the values distribution provide possibility for revealing bone, soft tissues and water in the picture.

It is the sixth aspect to provide estimation of inspected object material matrix part. Itis the mineral part of bone tissue that can be evaluated by longitudinal wave velocity in bone matrix.

The estimation is provided by applying statistical treatment of an obtained data for plurality elementary cells of an inspected object. The histogram of distribution of longitudinal wave velocity values helps to evaluate the value longitudinal wave velocity in the mineral part of the considered bone $V_t$ by the its maximum value. This value indirectly characterizes the mineral part of a considered bone medium for the concrete individual. It is the point of counting out of changes in bone substance for a concrete individual. The graph of the statistical treatment of the data is shown in the FIG. 13.

According to the graph the value $V_t$ = 5150 m/s may be taken as the longitudinal wave velocity in the matrix (skeleton) of the bone for the specimen, where bone tissue predominates.

The values of porosity n by the changed Wyllie relationship can be defined as known from geophysical literature with the aim of "Equation of average time". Itis expressed by longitudinal wave velocity values.

The equation is as follows: $\dfrac{1}{V_p} = \dfrac{1-n}{V_t} + \dfrac{n}{V_{fill}}$

The porosities values were calculated using the values corresponding elastic waves travel times in the considered cells of an inspected object $V_p$, in the matrix's part of the inspected medium $V_t$ and in the filling of the medium $V_{fill}$ (filling was water in the considered case).

Porosity was calculated for a plurality of elementary volumes of the specimen of pig's leg, with predominated bone part. Data from the table 3 was used.

The following equation is applied for calculating porosity values:

$$ n = \frac{(V_t - V_p)\, V_{fill}}{V_p\, (V_t - V_{fill})} $$

The porous medium in a bone substance is filled by marrow. Approximately the value velocity in the filling may be taken as equal to 1550 m/s in the case of the human body inspections.

The obtained porosity values are depicted in table 4.

A seventh aspect provides a possibility for complex estimations for bone inspections (different from not BMD) and a way to its evaluations. This possibility allows to estimate parameters associated with the bone material as well as with its structural properties and provides possibility for 3D imaging quantitative estimations of an inspected volume.

The proposed estimations for example for bone media are: bone porosity, longitudinal wave velocity, longitudinal wave velocity and porosity distribution in an inspected volume, longitudinal wave velocity in bone's skeleton (bone matrix part) by virtue of statistical treatment of an obtained data, evaluation risk of fracture value and its location by analysis of obtained maps and additional possibilities that the estimations provide.

FIG. 14 shows the porosity values distribution in the specimen 1 from pig's leg, which includes it's the main bone media. The experiments confirm the viability of the technology according to the current invention and its applications for diagnosing human body inspections as well as for technical uses. Additional experiments were performed using different types beef with bones. Results of inspections are presented as maps of obtained longitudinal wave velocities values and distributions of porosity values. The inspection results of the specimen of the beef including the bone are presented in the figures 7 (a, b) according to the proposed differential approach according to the invention.

The pig's leg specimen was evaluated before and after impacting it with few heavy blows and cut in the middle part of the specimen. Comparison of the obtained images "before" and "after" blows and cut is depicted in figures 15(a) and 15(b) respectively. FIG. 15(a) and 15(b) depict the maps of longitudinal wave velocity values distributions in the section of the inspected pig's leg. The cavities and crack are revealed by velocities values reduction from the considered comparison, as result of the blows and cut. The picture proposes the possibility revealing the sore zone in the obtained image. For example, we can to observe the crack appearance in the zone 12,8cm by fall up the value longitudinal wave velocity in the zone from 3400-3900m/s up to 2400-2900m/s by the images comparison. The crack appearance is results of impact.

FIG. 16 illustrates experimental results demonstrating 3D bone quantitative-imaging by integrating a number of 20 sections shifted along a third axis with the same step b=8mm. The pig's leg specimen was wrapped up with meat (muscle mass) for creation of the layered system. The aforesaid layered system was disposed into immersion liquid (water). The distributions of the longitudinal wave velocity are obtained as 2D images of the horizontal sections of the horizontal sections of the inspected specimen of pig leg after damages (the specimen underwent a few impact and cut in the middle part of the leg).

Repeatability of obtained results proves high reliability of the method.

FIG 17 schematically depicts an apparatus for inspection of organ such as a limb.

Three Dimensional Ultrasonic System 900 is used for evaluation of limb 920 includes a system consisting of different layers providing the needed condition for non-linear beams travel in an inspected object.

An optional "U" shaped solid frame 910 from system plates 911, 912, 914 providing acoustic impedance gradient to inspected object is used for supporting an inspected object Alternatively, the number plates may be changed according to considered object and considered patient. Alternatively, bottom system plates are placed on an examination table, the limb is placed above it and a top pillow 915 is used for covering the limb. Alternatively, only top pillow is used with or without a frame. Bottom system plats is not needed if examination of structures in the lower part (away from transducer 930) is not important. For example, the spinal structures of a patient may be examined with patient in prone position with only a top pillow on his back and without the use of a frame or system plats.

Infigure 17, a cross section of the examined limb schematically shows the skin 922, the soft tissue between the skin 922 and the bone 924 and the bone marrow 926.

A 2D transducers' array 93-0 is placed on top of the top pillow which interfaces between the curved limb and the flat array. Array 930 receives signals from, and transmits signals to controller 932 which comprises the elements depicted in figure 10.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub combination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims..

**Claims**

1. An imaging ultrasound tomography system for inspecting (900) a heterogeneous object,
   wherein said system comprises:

   (a) a three dimensional ultrasonic unit comprising

       (i) a grid of evenly spaced ultrasonic transducers (26) capable of transmitting an ultrasonic wave at an incident angle to said grid in response to excitation pulses, and capable of producing signals in response to ultrasonic waves received at an angle to said grid by other transducers of said grid; wherein said transducer grid is in acoustic contact with said inspected heterogeneous object; and
       (ii) a layered system adapted for accommodating the inspected heterogeneous object; said system comprising an acoustic impedance gradient causing the ultrasonic waves to propagate through said layered system and said inspected heterogeneous object along non-linear beam paths;

   (b) a signal generator (18) capable of generating short excitation pulses;
   (c) a scanning position controller (39) adapted for consecutively emitting said excitation pulses and directing said pulses to a selected transmitting transducer in said transducer grid and receiving signals created in other receiving transducers in response to said ultrasonic wave emitted by said transmitted transducer and propagated non-linearly through said inspected system according to a predetermined protocol;
   (d) a measuring time unit (21) capable of receiving said signals from said receiving transducers and measuring a time of wave travel between corresponding pairs of transmitting transducers and receiving transducers;
   (e) a processor (22) adapted for (i) acquiring a plurality of measured travel times corresponding to a plurality of paths between said transmitting and receiving transducers; (ii) calculating according to the differential approach a plurality of time values corresponding to a plurality of elementary cells composing the inspected heterogeneous object and calculating the lengths of travel of ultrasonic waves in said elementary cells; (iii) calculating values of longitudinal wave velocity and porosity corresponding to a plurality of travel times corresponding to a plurality of elementary cells; and, (iv) evaluating longitudinal wave velocity in a material matrix of said inspected heterogeneous object;
   (f) an image formation unit (24) adapted for:

       (i) two- and three-dimensional mapping distributions of said longitudinal wave velocity and porosity values in the inspected heterogeneous object volume;
       (ii) contouring a distinct area of the inspected heterogeneous object;

(iii) contouring a defective area at risk of object fracture; and
(iv) evaluating longitudinal wave velocity and porosity within said defective area
(v) estimating a fracture risk value for said inspected heterogeneous object;

**characterized in that**:

(a) said inspected heterogeneous object is divided into a plurality of elementary cells arranged in columns and rows in order to apply the differential approach;
(b) said processor is adapted to differentially calculate travel times corresponding to each elementary cell of said inspected heterogeneous object by means of the differential approach as follows:

i. calculating changes in travel times $\Delta t_m$ corresponding to each subsequent cell relative to the previous cell along each column by combining the average values $t_m$, $t_{m-1}$, $t_{dir}$, $t_{rec}$ from eight travel times of the direct and reciprocal directions according to equation: $\Delta t_m = t_m + t_{m-1} - t_{dir} - t_{rec}$ where $t_m$ is the average value between the longitudinal wave travel time for direct and reciprocal directions for the transducers arrangement from points m to point (-m), where m is a number of a transducer location; $t_{m-1}$ is an average value between longitudinal wave travel time for direct and reciprocal directions for transducers arrangement from point (m-1) to point (-m+1); $t_{dir}$ is an average value between longitudinal wave travel time for direct and reciprocal directions for transducers arrangement from point (-m) to point (m-1) and $t_{rec}$ is an average value between longitudinal wave travel time for direct and reciprocal directions for transducers arrangement from point m to point (-m+1);
ii. calculating a sequence of travel time values corresponding to each of said elementary cells of said column by summing said values of said changes in travel times in said elementary cells $\Delta\tau_m$ ($\Delta\tau_1$, $\Delta\tau_2$, $\Delta\tau_3$, $\Delta\tau_4$.... $\Delta\tau_m$) and the travel times in said previous elementary cell in said column $t_m$ ($t_1$, $t_2$, $t_3$, ...$t_m$) according to equation: $t_2 = t_1 + \Delta\tau_1$, $t_3 = t_2 + \Delta\tau_2$, $t_3 = t_2 + \Delta\tau_2$ ... and $t_m = t_{m-1} + \Delta\tau_{m-1}$;

(c) said processor is adapted to calculate the values of longitudinal wave velocity corresponding to each elementary cell by dividing a length of a beam's travel in an elementary cell by said travel time; wherein said length within said cells of columns is equal to 2b/sin$\alpha$ where b is a distance between said transducers in said grid and $\alpha$ is the incident angle;
(d) said processor is adapted to evaluate the value of longitudinal wave velocity in a material matrix portion of said inspected heterogeneous object by means of constructing a histogram of said obtained longitudinal wave velocity values corresponding to said plurality of said elementary cells and finding the maximum value of the longitudinal wave velocity from the histogram;
(e) said processor is adapted to calculate porosity values n for said plurality of said cells according to the following formula:

$$ n = \frac{(V_t - V_p)V_{fill}}{V_p(V_t - V_{fill})} $$

where $V_p$ is the longitudinal wave velocity in said elementary cell, $V_t$ is a longitudinal wave velocity in said material matrix portion, and $V_{fill}$ is a longitudinal wave velocity in a pore filling material.

2. The ultrasonic system of claim 1, wherein the processor calculates changes in travel times $\Delta t_m$ corresponding to each subsequent cell relative to the previous cell along each column by combining the average values $t_m$, $t_{m-1}$, $t_{dir}$, $t_{rec}$ from four travel times of the direct and reciprocal directions.

3. The ultrasonic system of claim 1, wherein the layered artificial system is formed from a group consisting of at least one plate, pillow, sleeve, and any combination thereof.

4. The ultrasonic system of claim 1, wherein the image formation unit is further capable of mapping the longitudinal wave velocity and porosity values distributions in a volume.

5. The ultrasonic system of claim 1, wherein the grid of ultrasonic transducers is adapted to transmit and receive the ultrasonic wave beams at a constant incident and receiving angle selected in the region from 0 up to 90 degrees relative to a vertical axis thereof.

6. The ultrasonic system of claim 1, wherein the ultrasonic waves have a length $\lambda_{wave}$ which is commensurable to a dimension d of the inspected heterogeneous object such that the inspected heterogeneous object in the layered system is considered as a local heterogeneity.

7. The system of claim 1, wherein the layered system is a natural layered system containing a heterogeneous object, said natural layered system being **characterized by** an acoustic impedance gradient causing the ultrasound waves to travel in the inspected heterogeneous object along non-linear paths.

8. The system of claim 1, wherein the image formation unit is adapted to integrate a number of 2D images performed along a third axis thereby providing the 3D image mapping distributions of longitudinal wave velocity and porosity values in the inspected heterogeneous object.

**Patentansprüche**

1. Bilderzeugungs-Ultraschalltomographiesystem zum Inspizieren (900) eines heterogenen Objekts, wobei das genannte System Folgendes umfasst:

a) eine dreidimensionale Ultraschalleinheit, die Folgendes umfasst:

i) ein Raster von gleichmäßig beabstandeten Ultraschallwandlern (26), die eine Ultraschallwelle mit einem Einfallswinkel auf das genannte Raster als Reaktion auf Anregungspulse senden und Signale als Reaktion auf Ultraschallwellen erzeugen kann, die in einem Winkel auf dem genannten Raster von anderen Wandlern des genannten Rasters empfangen werden; wobei das genannte Wandlerraster in akustischem Kontakt mit dem genannten inspizierten heterogenen Objekt ist; und
ii) ein mehrschichtiges System, ausgelegt zum Aufnehmen des inspizierten heterogenen Objekts; wobei das genannte System einen akustischen Impedanzgradienten umfasst, der bewirkt, dass sich die Ultraschallwellen durch das genannte mehrschichtige System und das genannte inspizierte heterogene Objekt entlang nichtlinearen Strahlpfaden ausbreiten;

b) einen Signalgenerator (18), der kurze Anregungspulse erzeugen kann;
c) einen Abtastpositionscontroller (39), ausgelegt zum konsekutiven Emittieren der genannten Anregungspulse und zum Leiten der genannten Pulse zu einem gewählten Sendewandler in dem genannten Wandlerraster und zum Empfangen von Signalen, die in anderen Empfangswandlern als Reaktion auf die von dem genannten gesendeten Wandler emittierte genannte Ultraschallwelle erzeugt wurden, die sich nichtlinear durch das genannte inspizierte System gemäß einem vorbestimmten Protokoll ausgebreitet hat;
d) eine Messzeiteinheit (21), die die genannten Signale von den genannten Empfangswandlern empfangen und eine Wellenwanderzeit zwischen entsprechenden Paaren von Sendewandlern und Empfangswandlern messen kann;
e) einen Prozessor (22), ausgelegt zum i) Erfassen von mehreren gemessenen Wanderzeiten entsprechend mehreren Pfaden zwischen den genannten Sende- und Empfangswandlern; ii) Berechnen, gemäß dem Differenzialansatz, von mehreren Zeitwerten entsprechend mehreren elementaren Zellen, aus denen das inspizierte heterogene Objekt zusammengesetzt ist, und Berechnen der Wanderlängen von Ultraschallwellen in den genannten elementaren Zellen; iii) Berechnen von Werten von Längswellengeschwindigkeit und Porösität entsprechend mehreren Wanderzeiten entsprechend mehreren elementaren Zellen; und iv) Beurteilen von Längswellengeschwindigkeit in einer Materialmatrix des genannten inspizierten heterogenen Objekts;
f) eine Bilderzeugungseinheit (24), ausgelegt zum:

i) zwei- und dreidimensionalen Mappen von Verteilungen der genannten Längswellengeschwindigkeit und Porösitätswerte in dem inspizierten heterogenen Objektvolumen;
ii) Konturieren eines bestimmten Bereichs des inspizierten heterogenen Objekts;
iii) Konturieren eines defekten Bereichs mit einem Objektbruchrisiko; und
iv) Beurteilen von Längswellengeschwindigkeit und Porösität in dem genannten defekten Bereich;
v) Schätzen eines Bruchrisikowertes für das genannte inspizierte heterogene Objekt;

**dadurch gekennzeichnet, dass**:

a) das genannte inspizierte heterogene Objekt in mehrere elementare Zellen unterteilt ist, die in Spalten und Reihen angeordnet sind, um den Differenzialansatz anzuwenden;

b) der genannte Prozessor zum differenzialen Berechnen von Wanderzeiten entsprechend jeder elementaren Zelle des genannten inspizierten heterogenen Objekts anhand des Differenzialansatzes wie folgt ausgelegt ist:

i) Berechnen von Änderungen der Wanderzeiten $\Delta t_m$ entsprechend jeder nachfolgenden Zelle relativ zur vorherigen Zelle entlang jeder Spalte durch Kombinieren der Durchschnittswerte $t_m$, $t_{m-1}$, $t_{dir}$, $t_{rec}$ von acht Wanderzeiten der Direkt- und Umkehrrichtungen gemäß der folgenden Gleichung: $\Delta t_m = t_m + t_{m-1} - t_{dir} - t_{rec}$, wobei $t_m$ der Durchschnittswert zwischen der Längswellenwanderzeit für Direkt- und Umkehrrichtungen für die Wandleranordnung von Punkt m zu Punkt (-m) ist, wobei m eine Nummer eines Wandlerortes ist; $t_{m-1}$ ein Durchschnittswert zwischen Längswellenwanderzeit für Direkt- und Umkehrrichtungen für eine Wandleranordnung von Punkt (m-1) zu Punkt (-m+1) ist; $t_{dir}$ ein Durchschnittswert zwischen Längswellenwanderzeit für Direkt- und Umkehrrichtungen für eine Wandleranordnung von Punkt (-m) zu Punkt (m-1) ist und $t_{rec}$ ein Durchschnittswert zwischen Längswellenwanderzeit für Direkt- und Umkehrrichtungen für eine Wandleranordnung von Punkt m zu Punkt (-m+1) ist;

ii) Berechnen einer Folge von Wanderzeitwerten entsprechend jeder der genannten elementaren Zellen der genannten Spalte durch Summieren der genannten Werte der genannten Änderungen von Wanderzeiten in den genannten elementaren Zellen $\Delta\tau_m$ ($\Delta\tau_1$, $\Delta\tau_2$, $\Delta\tau_3$, $\Delta\tau_4$ ... $\Delta\tau_m$) und der Wanderzeiten in der genannten vorherigen elementaren Zelle in der genannten Spalte $t_m$ ($t_1$, $t_2$, $t_3$, ... $t_m$) gemäß der Gleichung: $t_2 = t_1 + \Delta\tau_1$, $t_3 = t_2 + \Delta\tau_2$, $t_3 = t_2 + \Delta\tau_2$... und $t_m - t_{m-1} + \Delta\tau_{m-1}$ ;

c) der genannte Prozessor zum Berechnen der Werte von Längswellengeschwindigkeit entsprechend jeder elementaren Zelle durch Dividieren einer Länge einer Strahlwanderung in eine elementaren Zelle durch die genannte Wanderzeit ausgelegt ist; wobei die genannte Länge innerhalb der genannten Zellen von Spalten gleich $2b/\sin\alpha$ ist, wobei b eine Distanz zwischen den genannten Wandlern in dem genannten Raster und $\alpha$ der Einfallswinkel ist;

d) der genannte Prozessor zum Beurteilen des Wertes der Längswellengeschwindigkeit in einem Materialmatrixteil des genannten inspizierten heterogenen Objekts durch Konstruieren eines Histogramms der genannten erhaltenen Längswellengeschwindigkeitswerte entsprechend den genannten mehreren elementaren Zellen und Finden des maximalen Werts der Längswellengeschwindigkeit anhand des Histogramms ausgelegt ist;

e) der genannte Prozessor zum Berechnen von Porösitätswerten n für die genannten mehreren Zellen gemäß der folgenden Formel ausgelegt ist:

$$ n = \frac{(V_t - V_p)V_{fill}}{V_p(V_t - V_{fill})} $$

wobei $V_p$ die Längswellengeschwindigkeit in der genannten elementaren Zelle ist, $V_t$ eine Längswellengeschwindigkeit in dem genannten Materialmatrixteil ist und $V_{fill}$ eine Längswellengeschwindigkeit in einem Porenfüllmaterial ist.

2. Ultraschallsystem nach Anspruch 1, wobei der Prozessor Änderungen der Wanderzeiten $\Delta\tau_m$ entsprechend jeder nachfolgenden Zelle relativ zur vorherigen Zelle entlang jeder Spalte durch Kombinieren der Durchschnittswerte $t_m$, $t_{m-1}$, $t_{dir}$, $t_{rec}$ von vier Wanderzeiten der Direkt- und Umkehrrichtungen berechnet.

3. Ultraschallsystem nach Anspruch 1, wobei das mehrschichtige künstliche System von einer Gruppe bestehend aus wenigstens einer Platte, einem Kissen, einer Hülse und einer beliebigen Kombination davon gebildet ist.

4. Ultraschallsystem nach Anspruch 1, wobei die Bilderzeugungseinheit ferner die Längswellengeschwindigkeit und Porösitätswerteverteilungen in einem Volumen mappen kann.

5. Ultraschallsystem nach Anspruch 1, wobei das Raster von Ultraschallwandlern zum Senden und Empfangen der Ultraschallwellenstrahlen mit einem konstanten Einfalls- und Empfangswinkel ausgelegt ist, ausgewählt in der Region von 0 bis zu 90° relativ zu einer vertikalen Achse davon.

6. Ultraschallsystem nach Anspruch 1, wobei die Ultraschallwellen eine Länge $\lambda_{wave}$ haben, die mit einer Dimension d des inspizierten heterogenen Objekts im Einklang steht, so dass das inspizierte heterogene Objekt in dem mehrschichtigen System als lokale Heterogenität angesehen wird.

**7.** System nach Anspruch 1, wobei das mehrschichtige System ein natürliches mehrschichtiges System ist, das ein heterogenes Objekt enthält, wobei das genannte natürliche mehrschichtige System **dadurch gekennzeichnet ist, dass** ein akustischer Impedanzgradient bewirkt, dass die Ultraschallwellen in dem inspizierten heterogenen Objekt entlang nichtlinearen Pfaden wandern.

**8.** System nach Anspruch 1, wobei die Bilderzeugungseinheit zum Integrieren einer Anzahl von 2D-Bildern ausgelegt ist, durchgeführt entlang einer dritten Achse, um dadurch die 3D-Bild-Mapping-Verteilungen von Längswellengeschwindigkeit und Porösitätswerten in dem inspizierten heterogenen Objekt bereitzustellen.

**Revendications**

**1.** Système d'imagerie par tomographie à ultrasons destiné à inspecter (900) un objet hétérogène, dans lequel ledit système comprend :

(a) une unité ultrasonore tridimensionnelle comprenant

(i) une grille de transducteurs ultrasonores équidistants (26) capable de transmettre une onde ultrasonore à un angle d'incidence par rapport à ladite grille en réponse à des impulsions d'excitation, et capable de produire des signaux en réponse à des ondes ultrasonores reçues à un angle par rapport à ladite grille par d'autres transducteurs de ladite grille ; dans lequel ladite grille de transducteurs est en contact acoustique avec ledit objet hétérogène inspecté ; et
(ii) un système en couches adapté pour recevoir l'objet hétérogène inspecté ; ledit système comprenant un gradient d'impédance acoustique entraînant la propagation des ondes ultrasonores à travers ledit système en couches et ledit objet hétérogène inspecté le long de trajectoires de faisceau non linéaires ;

(b) un générateur de signal (18) capable de générer de courtes impulsions d'excitation ;
(c) un contrôleur de position de balayage (39) adapté pour émettre consécutivement lesdites impulsions d'excitation et dirigeant lesdites impulsions vers un transducteur de transmission sélectionné dans ladite grille de transducteurs et recevoir des signaux créés dans d'autres transducteurs de réception en réponse à ladite onde ultrasonore émise par ledit transducteur de transmission et propagée non linéairement à travers ledit système inspecté conformément à un protocole prédéterminé ;
(d) une unité de temps de mesure (21) capable de recevoir lesdits signaux depuis lesdits transducteurs de réception et de mesurer un temps de propagation d'ondes entre des paires correspondantes de transducteurs de transmission et de transducteurs de réception ;
(e) un processeur (22) adapté pour (i) acquérir une pluralité de temps de propagation mesurés correspondant à une pluralité de trajectoires entre lesdits transducteurs de transmission et de réception ; (ii) calculer conformément à l'approche différentielle une pluralité de valeurs temporelles correspondant à une pluralité de cellules élémentaires composant l'objet hétérogène inspecté et calculer les longueurs de propagation d'ondes ultrasonores dans lesdites cellules élémentaires ; (iii) calculer des valeurs de vitesse et de porosité d'ondes longitudinales correspondant à une pluralité de temps de propagation correspondant à une pluralité de cellules élémentaires ; et, (iv) évaluer une vitesse d'onde longitudinale dans une matrice de matériau dudit objet hétérogène inspecté ;
(f) une unité de formation d'image (24) adaptée pour :

(i) mapper en deux et trois dimensions des distributions desdites valeurs de vitesse et de porosité d'ondes longitudinales dans le volume d'objet hétérogène inspecté ;
(ii) tracer un contour d'une zone distincte de l'objet hétérogène inspecté ;
(iii) tracer un contour d'une zone défectueuse posant un risque de fracture d'objet ; et
(iv) évaluer une vitesse et une porosité d'ondes longitudinales dans ladite zone défectueuse ;
(v) estimer une valeur de risque de fracture dudit objet hétérogène inspecté ;

**caractérisé en ce que** :

(a) ledit objet hétérogène inspecté est divisé en une pluralité de cellules élémentaires agencées en colonnes et rangées afin d'appliquer l'approche différentielle ;
(b) ledit processeur est adapté pour calculer différentiellement des temps de propagation correspondant à chaque cellule élémentaire dudit objet hétérogène inspecté au moyen d'une approche différentielle comme suit :

i. en calculant des changements de temps de propagation $\Delta t_m$ correspondant à chaque cellule suivante relativement à la cellule précédente le long de chaque colonne en combinant les valeurs moyennes $t_m$, $t_{m-1}$, $t_{dir}$, $t_{rec}$ de huit temps de propagation des sens direct et réciproque conformément à l'équation : $\Delta t_m$ = $t_m$ + $t_{m-1}$ - $t_{dir}$ - $t_{rec}$ où $t_m$ est la valeur moyenne entre un temps de propagation d'onde longitudinale pour les sens direct et réciproque pour l'agencement de transducteurs des points m au point (-m), où m est un numéro d'emplacement de transducteur ; $t_{m-1}$ est une valeur moyenne entre un temps de propagation d'onde longitudinale pour les sens direct et réciproque d'un agencement de transducteurs du point (m-1) au point (-m+1) ; $t_{dir}$ est une valeur moyenne entre un temps de propagation d'onde longitudinale pour les sens direct et réciproque d'un agencement de transducteurs du point (-m) au point (m-1) et $t_{rec}$ est une valeur moyenne entre un temps de propagation d'onde longitudinale pour les sens direct et réciproque pour un agencement de transducteurs du point m au point (-m+1) ;

ii. en calculant une séquence de valeurs de temps de propagation correspondant à chacune desdites cellules élémentaires de ladite colonne en additionnant lesdites valeurs desdits changements de temps de propagation dans lesdites cellules élémentaires $\Delta \tau_m$ ($\Delta \tau_1$, $\Delta \tau_2$, $\Delta \tau_3$, $\Delta \tau_4$... $\Delta \tau_m$) et les temps de propagation dans ladite cellule élémentaire précédente dans ladite colonne $t_m$ ($t_1$, $t_2$, $t_3$, ... $t_m$) conformément à l'équation : $t_2 = t_1 + \Delta \tau_1$, $t_3 = t_2 + \Delta \tau_2$, $t_3 = t_2 + \Delta \tau_2$ ... et $t_m = t_{m-1} + \Delta \tau_{m-1}$ ;

(c) ledit processeur est adapté pour calculer les valeurs de vitesse d'onde longitudinale correspondant à chaque cellule élémentaire en divisant une longueur d'une propagation de faisceau dans une cellule élémentaire par ledit temps de propagation ; dans lequel ladite longueur dans lesdites cellules de colonnes est égale à 2b/sin$\alpha$ où b est une distance entre lesdits transducteurs dans ladite grille et $\alpha$ est l'angle d'incidence ;

(d) ledit processeur est adapté pour évaluer la valeur de la vitesse d'onde longitudinale dans une partie de matrice de matériau dudit objet hétérogène inspecté en construisant un histogramme desdites valeurs de vitesse d'onde longitudinale obtenues correspondant à ladite pluralité desdites cellules élémentaires et obtenant la valeur maximum de la vitesse d'onde longitudinale à partir de l'histogramme ;

(e) ledit processeur est adapté pour calculer des valeurs de porosité n pour ladite pluralité desdites cellules conformément à la formule suivante :

$$n \;=\; \frac{(V_t - V_p)V_{fill}}{V_p(V_t - V_{fill})}$$

où $V_p$ est la vitesse d'onde longitudinale dans ladite cellule élémentaire, $V_t$ est une vitesse d'onde longitudinale dans ladite partie de matrice de matériau, et $V_{fill}$ est une vitesse d'onde longitudinale dans un matériau de remplissage de pores.

2. Système à ultrasons selon la revendication 1, dans lequel le processeur calcule des changements de temps de propagation $\Delta t_m$ correspondant à chaque cellule suivante relativement à la cellule précédente le long de chaque colonne en combinant les valeurs moyennes $t_m$, $t_{m-1}$, $t_{dir}$, $t_{rec}$ de quatre temps de propagation des sens direct et réciproque.

3. Système à ultrasons selon la revendication 1, dans lequel le système artificiel en couches est formé à partir d'un groupe consistant en au moins une plaque, un coussin, un manchon, et n'importe quelle combinaison de ceux-ci.

4. Système à ultrasons selon la revendication 1, dans lequel l'unité de formation d'image est capable en outre de mapper les valeurs de vitesse et de porosité d'ondes longitudinales dans un volume.

5. Système à ultrasons selon la revendication 1, dans lequel la grille de transducteurs ultrasonores est adaptée pour transmettre et recevoir les faisceaux d'onde ultrasonore à un angle d'incidence et de réception constant sélectionné dans la région allant de 0 jusqu'à 90 degrés par rapport à un axe vertical de celle-ci.

6. Système à ultrasons selon la revendication 1, dans lequel les ondes ultrasonores ont une longueur $\lambda_{wave}$ qui est proportionnelle à une dimension d de l'objet hétérogène inspecté de telle sorte que l'objet hétérogène inspecté dans le système en couches soit considéré comme une hétérogénéité locale.

7. Système selon la revendication 1, dans lequel le système en couches est un système en couches naturel contenant un objet hétérogène, ledit système en couches naturel étant **caractérisé par** un gradient d'impédance acoustique amenant les ondes ultrasonores à se propager dans l'objet hétérogène inspecté le long de trajectoires non linéaires.

8. Système selon la revendication 1, dans lequel l'unité de formation d'image est adaptée pour intégrer un nombre d'images 2D exécutées le long d'un troisième axe fournissant ainsi les distributions de mappage d'image 3D des valeurs de vitesse et de porosité d'ondes longitudinales dans l'objet hétérogène inspecté.

**Fig 1**

Fig 2

Fig 3

**Fig 4**

Fig 5

Fig 6

Fig 7

$D_{1,n-1}$

$D_{1,n}$

(1)

(2)

$S_2$

$S_1$

26

$S_n$

19

(k-1)

(k)

29

27

# Fig 8

**Fig 9**

**Fig 10**

Fig 13

**Fig 11**

| Route | Distance between sensors | Measurement time in the route (-m) to (m). $\tau_m$ μs | | Measurement time in the route .(-m+1) to (m-1) $\tau_{m-1}$ μs | | Measurement time in the route (-m+1) to (m). $\tau_{dir}$ μs | | Measurement time in the route .(-m) to (m-1) $\tau_{rec}$ μs | |
|---|---|---|---|---|---|---|---|---|---|
| | cm | Direct $T_{m.dir}$ | Reciprocal $T_{m.rec}$ | Direct $T_{-m+1.dir}$ | Reciprocal $T_{-m+1.rec}$ | Direct $T_{dir.dir}$ | Reciprocal $T_{dir.rec}$ | Direct $T_{rec.dir}$ | Reciprocal $T_{rec.rec}$ |
| 1 | 2 | 3 | 4 | 5 | 6 | 7 | 8 | 9 | 10 |
| **Section 10.8cm** | | | | | | | | | |
| 5to32 | 10.8 | 172 | 172 | 162 | 160 | 168 | 167 | 169 | 169 |
| 6to31 | 10 | 162 | 160 | 151 | 153 | 157 | 157 | 157 | 156 |
| 7to30 | 9.2 | 151 | 153 | 142 | 140 | 146 | 143 | 147 | 147 |
| 8to29 | 8.4 | 142 | 140 | 129 | 132 | 137 | 137 | 136 | 136 |
| 9to28 | 7.6 | 129 | 132 | 111 | 122 | 113 | 126 | 118 | 121 |
| 10to27 | 6.8 | 111 | 122 | 100 | 107 | 103 | 109 | 100 | 107 |
| 11to26 | 6 | 100 | 107 | 92 | 93 | 96 | 99 | 98 | 96 |
| 12to25 | 5.2 | 92 | 93 | 89 | 88 | 89 | 88 | 88 | 89 |
| **Section 10cm** | | | | | | | | | |
| 5to30 | 10 | 161 | 163 | 152 | 152 | 157 | 156 | 157 | 158 |
| 6to29 | 9.2 | 152 | 152 | 140 | 143 | 147 | 147 | 147 | 148 |
| 7to28 | 8.4 | 140 | 143 | 130 | 132 | 136 | 136 | 137 | 136 |
| 8to27 | 7.6 | 130 | 132 | 111 | 118 | 118 | 121 | 121 | 127 |
| 9to26 | 6.8 | 111 | 118 | 102 | 102 | 100 | 107 | 106 | 112 |
| 10to25 | 6 | 102 | 102 | 92 | 91 | 98 | 96 | 103 | 96 |
| 11to24 | 5.2 | 92 | 91 | 88 | 86 | 88 | 89 | 86 | 86 |
| **Section 9.2cm** | | | | | | | | | |
| 5to28 | 9.2 | 151 | 151 | 141 | 141 | 147 | 148 | 147 | 138 |
| 6to27 | 8.4 | 141 | 141 | 131 | 131 | 137 | 136 | 136 | 136 |
| 7to26 | 7.6 | 131 | 131 | 112 | 120 | 121 | 127 | 122 | 126 |
| 8to25 | 6.8 | 112 | 120 | 106 | 108 | 106 | 112 | 112 | 111 |
| 9to24 | 6 | 106 | 108 | 92 | 90 | 103 | 96 | 100 | 102 |
| 10to23 | 5.2 | 92 | 90 | 88 | 87 | 86 | 86 | 86 | 86 |

# Table 1

| Distance, between transmitter and receiver, cm | $\Delta t$, $\mu s$ according to the algorithm | | | $t_m$, $\mu s$ (15,29+$\Delta t$) | | | Longitudinal wave velocity, V, m/s s/ $t_m$ | | |
|---|---|---|---|---|---|---|---|---|---|
| | Section, cm | | | | | | | | |
| | 10,8 | 10 | 9,2 | 10,8 | 10 | 9,2 | 10,8 | 10 | 9,2 |
| 10,8 | -3,5 | 0 | 2 | 11,8 | 15,3 | 17,3 | 2129 | 1642 | 1452 |
| 10 | -0,5 | -1 | -0,5 | 14,8 | 14,3 | 14,8 | 1697 | 1757 | 1697 |
| 9,2 | 1,5 | 0 | -1 | 16,8 | 15,3 | 14,3 | 1495 | 1641 | 1757 |
| 8,4 | -1,5 | 2 | 2,5 | 13,8 | 17,3 | 17,8 | 1820 | 1452 | 1411 |
| 7,6 | 8 | 4 | -2,5 | 23,3 | 19,3 | 12,8 | 1078 | 1301 | 1963 |
| 6,8 | 10,5 | -3 | 6,5 | 25,8 | 12,3 | 21,8 | 973 | 2042 | 1152 |
| 6 | 1,5 | 4 | | 16,8 | 19,3 | | 1495 | 1302 | |
| 5,2 | 4 | | | 19,3 | | | 1301 | | |

# Table 2

| The longitudinal wave velocity values, m/s | | | | | |
|---|---|---|---|---|---|
| Distance, cm | Distance, cm | | | | |
| | 4,5 | 5,3 | 6,1 | 6,9 | 7,7 |
| 10,4 | 3213 | 2834 | 3443 | 2534 | 4388 |
| 11,2 | 3213 | 3709 | 2407 | 3213 | 2407 |
| 12 | 3443 | 3709 | 3443 | 3709 | 3011 |
| 12,8 | 3709 | 2834 | 2092 | 28334 | 2834 |
| 13,6 | 3709 | 2834 | 2534 | 4020 | 4388 |
| 14,4 | 2834 | 3213 | 4510 | 4490 | 3443 |
| 15,2 | 1718 | 1603 | 2092 | 4490 | 2005 |
| 16 | 2534 | 3011 | 3709 | 2407 | 3213 |
| 16,8 | 2407 | 2834 | 3443 | 3213 | 4388 |
| 17,6 | 3011 | 3443 | 4020 | 2834 | 1659 |
| 18,4 | 4388 | 2676 | 2534 | 2834 | 3213 |
| 19,2 | 2534 | 4020 | 3011 | 2834 | 4020 |

# Table 3

air
☐ 200-500

water+air
☐ 500-800  ⊞ 800-1100

tissues
☐ 1100-1400  ⊞ 1400-1700

bone
☐ 1700-2000  ⊞ 2000-2300  ☐ 2300-2600  ☐ 2600-2900

m/s

Fig 12

| Porosity values | | | | | |
|---|---|---|---|---|---|
| Distance, Between sensors, cm | 4,5 | 5,3 | 6,1 | 6,9 | 7,7 |
| 10,4 | 0,260 | 0,352 | 0,213 | 0,444 | 0,075 |
| 11,2 | 0,260 | 0,167 | 0,491 | 0,260 | 0,491 |
| 12 | 0,213 | 0,167 | 0,213 | 0,167 | 0,306 |
| 12,8 | 0,167 | 0,352 | 0,629 | 0,352 | 0,352 |
| 13,6 | 0,167 | 0,352 | 0,444 | 0,121 | 0,075 |
| 14,4 | 0,352 | 0,260 | 0,061 | 0,063 | 0,213 |
| 15,2 | 0,860 | 0,952 | 0,629 | 0,063 | 0,675 |
| 16 | 0,444 | 0,306 | 0,167 | 0,491 | 0,260 |
| 16,8 | 0,491 | 0,352 | 0,213 | 0,260 | 0,075 |
| 17,6 | 0,306 | 0,213 | 0,121 | 0,352 | 0,906 |
| 18,4 | 0,075 | 0,398 | 0,444 | 0,352 | 0,260 |
| 19,2 | 0,444 | 0,121 | 0,306 | 0,352 | 0,121 |

# Table 4

Fig 14

EP 2 077 759 B1

(a)

(b)

**Fig 15**

Fig. 16

**Fig 17**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6517487 B
- US 20030055334 A
- US 20030055337 A
- US 20040193047 A
- US 20050033140 A
- US 20050049479 A
- US 20050124886 A
- US 20050117694 A
- US 20030018263 A
- US 20050107700 A
- US 6305060 B
- US 6012779 A
- US 6371916 B
- US 4926870 A
- US 4421119 A
- US 20020134178 A, Knight
- US 6221019 B
- US 5143072 A

### Non-patent literature cited in the description

- **LANGTON C M ; PALMER S B ; PORTER R W.** The Measurement of Broadband Ultrasonic Attenuation in Trabecular Bone. *Engineering in Medicine,* 1984, vol. 13 (3), 89-91
- **WYLLIE, M.R.** An experimental investigation of factors affecting elastic wave velocities in porous media. *Geophysics,* 1958, vol. 23 (3
- Ore seismic. **N.A.KARAEV ; CI.YA. RABINOVICH.** Geoinfonnmark Closed Joint. Stock Company, 2000, vol. ill, 366
- **L.A.PEVZNER ; V.L.POKIDOV ; V.A.TSIMER.** The seismic investigation of complex medium. *Alma-Ata. Science,* 1984